# EUROPEAN PATENT APPLICATION

(11) **EP 3 822 637 A1**
(43) Date of publication of application: **19.05.2021**
(21) Application number: 19208577.7
(22) Date of filing: 12.11.2019
(51) Int. Cl.: G01N 33/574

(54) **METHOD FOR DETERMINING PARP INHIBITOR RESPONSIVENESS AND IMPROVING PARP INHIBITOR THERAPY**

(71) Applicant: Universität Zürich, 8006 Zürich (CH)
(72) Inventor: The designation of the inventor has not yet been filed
(74) Representative: Schulz Junghans Patentanwälte PartGmbB

(57) **Abstract**

The invention relates to a method for determining the responsiveness of a patient's tumour disease to treatment by administration of a PARP inhibitor. The method comprising the steps of determining a level of TRIP12 protein and/or a level of an mRNA encoding TRIP12 protein, or the presence of a TRIP12 mutation, and comparing the TRIP12 expression level and/or the TRIP12 activity level with a threshold value, and assigning to said tumour disease a likelihood of responsiveness.

The invention further relates to a PARP inhibitor for use in treatment of a tumour disease associated with a level of TRIP12 expression lower than a threshold, or to a PARP inhibitor for use in treatment of a tumour disease administered concomitant with or after administration of an ubiquitin ligase inhibitor and/or a nucleic acid agent capable of decreasing or inhibiting or blocking TRIP12 expression.

## Description

The invention relates to a method for determining the responsiveness of a patient's tumour disease to treatment by administration of a PARP inhibitor. The invention further relates to a PARP inhibitor for use in treatment of a tumour disease.

### Background of the Invention

Poly(ADP-ribose) polymerase 1 (PARP1) is an abundant chromatin-associated enzyme, whose activity is strongly induced in response to genotoxic stress. PARP1 has affinity for DNA single- and double-strand breaks, for stalled and reversed replication forks, and for a range of atypical DNA secondary structures. Upon DNA binding and allosteric activation, PARP1 uses the energy carrier nicotinamide adenine dinucleotide (NAD+) as substrate to generate poly(ADP-ribose) (PAR) chains. PAR formation induces liquid-liquid phase separation of intrinsically disordered PAR-binding proteins at chromosome break sites and is important for the timely recruitment of PAR-binding repair factors and for rearranging the local chromatin structure in response to DNA damage. PAR signalling thus promotes DNA repair reactions and helps maintain genome stability. When genotoxic stress is too severe, however, sustained PARylation depletes the cellular NAD+ pool and causes a PAR-dependent type of cell death known as PARthanatos.

While PARP inhibitors (PARPi) protect cells from PARP1-mediated NAD+ depletion, their clinical use is primarily associated with causing synthetic lethality in BRCA1/2-deficient tumors. The tumour suppressors BRCA1 and BRCA2 are critical components of the homology-directed repair (HDR) pathway, which promotes error-free repair of DNA double-strand breaks (DSBs) and emerges as an important contributor to replication fork stability. In a fraction of heritable and sporadic breast and ovarian cancers BRCA1/2 function is lost, and this situation generates a cancer-specific vulnerability to PARPi. Based on the promising pre-clinical and clinical results in BRCA-deficient contexts, current efforts aim at identifying signatures of HDR dysfunction, so that PARPi therapies could be used in HDR-defective settings and potentially beyond BRCA mutations.

Vulnerabilities to PARPi may, however, also exist in HDR-proficient cancer cells. In fact, by blocking access of NAD+ to the catalytic site, PARPi lock PARP1 in an inactive cytotoxic conformation, a situation generally referred to as PARP trapping. Consistent with PARP trapping being a major cause of PARPi toxicity in BRCA-proficient cells, loss of PARP1 rescues from PARPi-induced genotoxic stress and cell death.

Based on the above-mentioned state of the art, the objective of the present invention is to provide means and methods to identify novel diagnostic criteria for likely PARP inhibitor responsiveness, and to identify novel uses for PARP inhibitor pharmaceuticals.

This objective is attained by the subject-matter of the independent claims of the present specification.

The inventors reasoned that when PARPi are applied in conditions of BRCA1/2 and HDR proficiency, it is neither the impaired PARP1 function nor the inability to produce PAR, but rather the inactivated trapped PARP1 itself, which becomes detrimental for cell survival. Cellular regulators of PARP1 levels and/or PARP trapping may thus determine the efficiency of PARPi, and uncovering such regulators and characterizing their molecular and cellular functions opens new possibilities for cancer patients both with and without a BRCAness signature to potentially benefit from PARPi therapy.

### Summary of the Invention

A first aspect of the invention relates to a method for determining the responsiveness of a patient's tumour disease to treatment by administration of a PARP inhibitor. This method comprises the steps of
a. determining in a TRIP12 status determination step, in a sample having been obtained from the patient,
   i. a level of TRIP12 protein and/or
   ii. a level of an mRNA encoding TRIP12 protein
      wherein said level is designated a TRIP12 expression level
      and/or
   iii. the presence of a TRIP12 mutation or of a pathological alteration of TRIP12 function, yielding a TRIP12 activity level,
      and
b. comparing said TRIP12 expression level and/or said TRIP12 activity level with a threshold value,
c. assigning to said tumour disease a likelihood of responsiveness.

A second aspect of the invention relates to a pharmaceutical composition comprising a PARP inhibitor for use in treatment of a tumour disease, wherein the tumour disease is associated with tumour cells characterized by a level of TRIP12 expression lower than a threshold and/or TRIP12 activity lower than a threshold.

An alternative of this aspect relates to a pharmaceutical composition comprising a PARP inhibitor for use in treatment of a tumour disease, wherein the pharmaceutical composition is administered before, concomitant with or after administration of
a. a ubiquitin ligase inhibitor, particularly a HECT ubiquitin ligase inhibitor, more particularly Heclin, and/or
b. a nucleic acid agent capable of decreasing or inhibiting or blocking TRIP12 expression.
c. a peptide agent capable of decreasing or inhibiting or blocking TRIP12 activity or a small molecule agent capable of decreasing or inhibiting or blocking TRIP12 activity.

### Brief Description of the Figures

- Fig. 1: The HECT-type ubiquitin ligase TRIP12 controls PARP1 abundance.
- Fig. 2: TRIP12 limits PARylation and affects outcomes of PAR signaling.
- Fig. 3: TRIP12 interacts with and poly-ubiquitylates PARP1 in a PAR- and WWE-dependent manner.
- Fig. 4: TRIP12 controls PARP1 stability and proteasomal degradation in a PAR-dependent manner.
- Fig. 5: TRIP12 constrains PARP1 trapping and extenuates PARPi-induced DNA damage.
- Fig. 6: Down-regulation of TRIP12 sensitizes cancer cells to PARPi and is associated with elevated PARP1 abundance in cancer patients.
- Fig. 7: The HECT-type ubiquitin ligase TRIP12 controls PARP1 abundance.
- Fig. 8: TRIP12 limits cellular PAR formation.
- Fig. 9: PAR- and WWE-dependent interaction between PARP1 and TRIP12.
- Fig. 10: TRIP12 controls PARP1 stability.
- Fig. 11: TRIP12 constrains PARP1 trapping and extenuates PARPi-induced DNA damage.
- Fig. 12: Down-regulation of TRIP12 sensitizes cancer cells to PARPi.
- Fig. 13: Low TRIP12 expression is associated with elevated PARP1 abundance in cancer patients.
- Fig. 14: Elevated PARP1 levels sensitizes cancer cells to PARP inhibition.
- Fig. 15: A partial defect in TRIP12 sensitizes cancer cells to the PARP inhibitor talazoparib.
- Fig. 16: The HECT ubiquitin ligase inhibitor Heclin (CAS Number 890605-54-6) inhibits TRIP12 in vitro and in vivo and synergizes with PARP inhibition to kill cancer cells.

### Detailed Description of the Invention

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc.) and chemical methods.

The term *gene* refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The terms *capable of forming a hybrid* or *hybridizing sequence* in the context of the present specification relate to sequences that under the conditions existing within the cytosol of a mammalian cell, are able to bind selectively to their target sequence. Such hybridizing sequences may be contiguously reverse-complimentary to the target sequence, or may comprise gaps, mismatches or additional non-matching nucleotides. The minimal length for a sequence to be capable of forming a hybrid depends on its composition, with C or G nucleotides contributing more to the energy of binding than A or T/U nucleotides, and the backbone chemistry.

The term *Nucleotides* in the context of the present specification relates to nucleic acid or nucleic acid analogue building blocks, oligomers of which are capable of forming selective hybrids with RNA or DNA oligomers on the basis of base pairing. The term *nucleotides* in this context includes the classic ribonucleotide building blocks adenosine, guanosine, uridine (and ribosylthymine), cytidine, the classic deoxyribonucleotides deoxyadenosine, deoxyguanosine, thymidine, deoxyuridine and deoxycytidine. It further includes analogues of nucleic acids such as phosphotioates, 2'O-methylphosphothioates, *peptide nucleic acids* (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). Wherever reference is made herein to a *hybridizing sequence,* such hybridizing sequence may be composed of any of the above nucleotides, or mixtures thereof.

The term *siRNA* (small/short interfering RNA) in the context of the present specification relates to an RNA molecule capable of interfering with the expression (in other words: inhibiting or preventing the expression) of a gene comprising a nucleic acid sequence complementary or hybridizing to the sequence of the siRNA in a process termed RNA interference. The term *siRNA* is meant to encompass both single stranded siRNA and double stranded siRNA. siRNA is usually characterized by a length of 17-24 nucleotides. Double stranded siRNA can be derived from longer double stranded RNA molecules (dsRNA). According to prevailing theory, the longer dsRNA is cleaved by an endo-ribonuclease (called Dicer) to form double stranded siRNA. In a nucleoprotein complex (called RISC), the double stranded siRNA is unwound to form single stranded siRNA. RNA interference often works via binding of an siRNA molecule to the mRNA molecule having a complementary sequence, resulting in degradation of the mRNA. RNA interference is also possible by binding of an siRNA molecule to an intronic sequence of a pre-mRNA (an immature, non-spliced mRNA) within the nucleus of a cell, resulting in degradation of the pre-mRNA.

The term *shRNA* (small hairpin RNA) in the context of the present specification relates to an artificial RNA molecule with a tight hairpin turn that can be used to silence target gene expression via RNA interference (RNAi).

The term *sgRNA* (single guide RNA) in the context of the present specification relates to an RNA molecule capable of sequence-specific repression of gene expression via the CRISPR (clustered regularly interspaced short palindromic repeats) mechanism.

The term *miRNA* (microRNA) in the context of the present specification relates to a small noncoding RNA molecule (containing about 22 nucleotides) that functions in RNA silencing and post-transcriptional regulation of gene expression.

The term *antisense oligonucleotide* in the context of the present specification relates to an oligonucleotide having a sequence substantially complimentary to, and capable of hybridizing to, an RNA. Antisense action on such RNA will lead to modulation, particular inhibition or suppression of the RNA's biological effect. If the RNA is an mRNA, expression of the resulting gene product is inhibited or suppressed. Antisense oligonucleotides can consist of DNA, RNA, nucleotide analogues and/or mixtures thereof. The skilled person is aware of a variety of commercial and non-commercial sources for computation of a theoretically optimal antisense sequence to a given target. Optimization can be performed both in terms of nucleobase sequence and in terms of backbone (ribo, deoxyribo, analogue) composition. Many sources exist for delivery of the actual physical oligonucleotide, which generally is synthesized by solid state synthesis.

The term *nucleic acid expression vector* in the context of the present specification relates to a plasmid or a viral genome, which is used to transfect (in case of a plasmid) or transduce (in case of a viral genome) a target cell with a certain gene of interest. The gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

In certain embodiments, the hybridizing sequence of the nucleic acid agent according to the invention comprises 8, 9, 10, 11, 12, 13, 14, 15, 16, 17, 18, 19, 20, 21, 22, 23, 24, 25, 26, 27, 28, 29 or 30 nucleotides.

In the context of the present specification, the term *hybridizing sequence* encompasses a polynucleotide sequence comprising or essentially consisting of RNA (ribonucleotides), DNA (deoxyribonucleotides), phosphothioate deoxyribonucleotides, 2'-O-methyl-modified phosphothioate ribonucleotides, LNA and/or PNA nucleotide analogues.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

A first aspect of the invention relates to a method for determining, predicting or assessing, the responsiveness of a patient's tumour disease to treatment by administration of a PARP inhibitor to the patient. This method comprises the steps of
a. determining, in a sample having been obtained from the patient,
   i. a level of TRIP12 protein and/or
   ii. a level of an mRNA encoding TRIP12 protein
      wherein said level is designated a TRIP12 expression level
      and/or
   iii. the presence of a TRIP12 mutation, or of a pathological alteration of TRIP12 function, that decreases or abrogates TRIP12 biological activity, yielding a TRIP12 activity level,
   in a TRIP12 status determination step and
b. comparing said TRIP12 expression level and/or said TRIP12 activity level with a threshold value,
c. assigning to said tumour disease a likelihood of responsiveness.

The TRIP12 expression level can be determined both with regard to the amount of protein per cell or sample, and with regard to the amount of mRNA.

The TRIP12 status determination step quantifies the biological presence either as TRIP12 gene copy number or amount of protein or protein-encoding mRNA, or by determining TRIP12 functionality in a functional assay, or by genomic sequencing if TRIP12 malfunction or absence is to be predicted from sequencing data.

The determination of the level of TRIP12 protein can be achieved by determination of absolute amounts of TRIP12, or by determination of the amount relative to a standard. The determation or measurement of an amount of TRIP12 protein can be achieved by appropriate means such as Flow Cytometry and/or Western blot and/or immunohistochemistry. In certain embodiments, determining a level of TRIP12 protein comprises determining / measuring the amount of biological activity of TRIP12 protein by appropriate means such as enzymatic activity and/or protein interaction assays.

In certain embodiments, the TRIP12 expression level and/or TRIP12 activity level is compared with / normalized in relation to an expression level of a housekeeping gene in the patient tumour sample. Herein, the expression level of a housekeeping gene is or defines the threshold level. The inventors have successfully employed RPS12 and EIF2C2 as internal references for this purpose. Other examples include, but are not limited to, ACTB, HPRT1, TBP, GAPDH and RPL19.

In certain embodiments, the TRIP12 expression level and/or TRIP12 activity level is compared with an expression level of TRIP12 in a sample different from the patient sample tumour sample. One example of such reference or control sample is a sample of non-disease-related tissue from the same patient. If no other information is given, reference to a control or threshold made in here (i.e. "control TRIP12 protein and/or mRNA expression level and/or TRIP12 activity level") is to be understood as being made in comparison to a sample of non-disease-related tissue from the same patient.

Another example is a sample, or a collection of samples or cells, from tumours of other patients. Any such sample can be employed as a control.

The TRIP12 expression level or TRIP12 activity level in the control can be used directly as the threshold level. Then, the two values are simply the same.

Similarly, the TRIP12 expression level or TRIP12 activity in the control can define the threshold level without being equal to it. In this case, a certain calculation factor ("fold change") can be applied to the reference or control to determine whether the sample level is above or below the threshold.

In certain embodiments, the TRIP12 expression level and/orTRIP12 activity level of the patient sample is compared to the average or median TRIP12 expression level and/or TRIP12 activity level determined for reference tumour cells of the same tissue origin or similar tissue origin from a group of patients (one embodiment of a "reference"). In other words, the TRIP12 expression level and/or TRIP12 activity level of the patient sample is compared to a value derived from a panel of comparable tumours of other patients. These can be tumours sampled without knowledge of their TRIP12 status or PARP inhibitor responsiveness status, thus giving only a positionof the tumour's respective stati compared to a representative "whole" of tumours of the same tissue. Alternatively, the TRIP12 expression level and/or TRIP12 activity level of the patient sample can be compared to a value averaging a number of tumour samples from other patients that have known values with respect to their TRIP12 status or PARP inhibitor responsiveness status, either high or low.

In certain embodiments, the TRIP12 expression level and/or TRIP12 activity level is compared to the average or median TRIP12 expression level and/or TRIP12 activity level determined for reference tumour cells of the same tissue origin or similar tissue origin (another embodiment of a "reference"), wherein the reference tumour cells have been determined to be resistant to PARP inhibitor treatment.

In certain embodiments, the TRIP12 expression level and/or TRIP12 activity level is compared to the average or median TRIP12 expression level and/or TRIP12 activity level determined for reference tumour cells of the same or similar tissue origin, wherein the reference tumour cells have been determined to be responsive to PARP inhibitor treatment, or have been determined to be unresponsive to PARP inhibitor treatment. Herein, the TRIP12 expression level and/or TRIP12 activity level determined for reference tumour cells of the same or similar tissue origin is or defines the threshold level.

In certain embodiments, the TRIP12 expression level and/or TRIP12 activity level is compared to the average or median TRIP12 expression level and/or TRIP12 activity level of cells obtained from healthy tissue of the patient. Herein, the TRIP12 expression level and/or TRIP12 activity level determined for reference tumour cells of the same or similar tissue origin is or defines the threshold level.

In certain embodiments, a high, or above average, or above control likelihood of responsiveness is assigned to said tumour disease if said TRIP12 expression level and/or TRIP12 activity level is below said threshold value.

In certain embodiments, the TRIP12 expression level is determined by quantitative PCR.

In certain embodiments, the TRIP12 (protein) expression level is determined by PARP12 Western blot.

In certain embodiments, the TRIP12 expression level is determined by Flow Cytometry.

In certain embodiments, the TRIP12 (protein) expression level is determined by immunohistochemistry.

In certain embodiments, the presence or activity of TRIP12 is determined by genomic sequencing of the TRIP12 encoding gene.

In certain embodiments, the presence or activity of TRIP12 is determined by measuring the copy number of the TRIP12 encoding gene.

In certain embodiments, the TRIP12 activity is determined by TRIP12 activity assay in a tumour sample and/or in tumour-derived material.

Ideally, the determination of TRIP12 expression and activity reveals conclusively whether the tumour is amenable to PARP inhibitor therapy.

In certain embodiments, the threshold is about 60% or less of control TRIP12 protein and/or mRNA expression level and/or TRIP12 activity level.

In certain embodiments, the threshold is about 55% or less of control TRIP12 protein and/or mRNA expression level and/or TRIP12 activity level.

In certain embodiments, the threshold is about 50% or less of control TRIP12 protein and/or mRNA expression level and/or TRIP12 activity level.

In certain embodiments, the threshold is about 45% or less of control TRIP12 protein and/or mRNA expression level and/or TRIP12 activity level.

In certain embodiments, the threshold is about 40% or less of control TRIP12 protein and/or mRNA expression level and/or TRIP12 activity level.

In certain embodiments, the threshold is about 35% or less of control TRIP12 protein and/or mRNA expression level and/or TRIP12 activity level.

In certain embodiments, the threshold is about 30% or less of control TRIP12 protein and/or mRNA expression level and/or TRIP12 activity level.

In certain embodiments, the threshold is about 25% or less of control TRIP12 protein and/or mRNA expression level and/or TRIP12 activity level.

In certain embodiments, the threshold is about 20% or less of control TRIP12 protein and/or mRNA expression level and/or TRIP12 activity level.

In certain embodiments, the threshold is about 15% or less of control TRIP12 protein and/or mRNA expression level and/or TRIP12 activity level.

In certain embodiments, the threshold is about 10% or less of control TRIP12 protein and/or mRNA expression level and/or TRIP12 activity level.

In certain embodiments, the threshold is about 5% or less of control TRIP12 protein and/or mRNA expression level and/or TRIP12 activity level.

For the purpose of determining TRIP12 expression threshold levels, quantitative mRNA shall be used unless explicitly stated otherwise.

In certain embodiments, the PARP inhibitor inhibits PARP1. In certain embodiments, the PARP inhibitor inhibits PARP2. In certain embodiments, the PARP inhibitor inhibits both PARP1 and PARP2.

In certain embodiments, the PARP inhibitor is Olaparib (Lynparza®, CAS No. 763113-22-0). In certain embodiments, the PARP inhibitor is Rucaparib (Rubraca®, CAS No. 283173-50-2). In certain embodiments, the PARP inhibitor is Talazoparib (Talzenna®, CAS No CAS No. 1207456-01-6). In certain embodiments, the PARP inhibitor is Veliparib (ABT-888; CAS No 912444-00-9). In certain embodiments, the PARP inhibitor is Niraparib (Nejula®, CAS No 1038915-60-4). In certain embodiments, the PARP inhibitor is Pamiparib (CAS No.: 1446261-44-4). In certain embodiments, the PARP inhibitor is CEP-9722 (11-methoxy-2-((4-methylpiperazin-1-yl)methyl)-4,5,6,7-tetrahydro-1H-cyclopenta[a]pyrrolo[3,4-c]carbazole-1,3(2H)-dione; CAS#: 916574-83-9).

In certain embodiments, the tumour disease is breast cancer.

In certain embodiments, the tumour disease is ovarian cancer.

In certain embodiments, the tumour disease is prostate cancer.

In certain embodiments, the tumour disease is pancreas cancer.

In certain embodiments, the tumour disease is glioblastoma.

In certain embodiments, the tumour disease is lung cancer.

In certain embodiments, the tumour disease is colon cancer.

In certain embodiments, the tumour disease is selected from leukemia and lymphoma.

In certain embodiments, the tumour disease is characterized by presence of a somatic mutation or germ line mutation leading to gene BRCA deficiency or BRCA mutation, or leading to HDR deficiency in the tumour.

In certain embodiments, the tumour disease is characterized by absence of a somatic mutation or germ line mutation leading to gene BRCA deficiency or BRCA mutation, or HDR deficiency in said tumour. In other words, TRIP12 status determination can facilitate the use of PARP inhibitors in patients for whom such treatment has hitherto not been indicated.

Another aspect of the invention relates to a pharmaceutical composition comprising a PARP inhibitor for use in treatment of a tumour disease, wherein the tumour disease is associated with, or caused by, tumour cells characterized by a level of TRIP12 protein and/or mRNA expression and/or activity lower than a threshold.

In certain embodiments, the tumour disease is characterized by presence of a somatic mutation or germ line mutation resulting in TRIP12 protein level and/or mRNA expression level and/or activity level lower than a threshold.

In certain embodiments, the threshold is 50% of control TRIP12 protein level and/or mRNA expression level and/or TRIP12 activity level compared to a control selected from healthy patients and non-cancerous cells of the same patient.

In certain embodiments, the threshold is 40% of control TRIP12 protein level and/or mRNA expression level and/or TRIP12 activity level compared to a control selected from healthy patients and non-cancerous cells of the same patient.

In certain embodiments, the threshold is 30% of control TRIP12 protein level and/or mRNA expression level and/or TRIP12 activity level compared to a control selected from healthy patients and non-cancerous cells of the same patient.

In certain embodiments, the threshold is 55%, 50%, 45%, 40%, 35%, 30%, 25%, 20%, 15%, 10%, or 5% of control TRIP12 protein level and/or mRNA expression level and/or TRIP12 activity level compared to a control selected from healthy patients and non-cancerous cells of the same patient.

Yet another aspect of the invention relates to a pharmaceutical composition comprising a PARP inhibitor for use in treatment of a tumour disease, wherein the pharmaceutical composition is administered before, concomitant with or after administration of
a. a ubiquitin ligase inhibitor, particularly a HECT ubiquitin ligase inhibitor, more particularly Heclin, and/or
b. a nucleic acid agent capable of decreasing or inhibiting or blocking TRIP12 expression.

Co-administration of PARP inhibitors and ubiquitin ligase inhibitors can facilitate PARP inhibitor therapies to patients who are unlikely to be responsive to PARP inhibitor therapy alone.

In certain embodiments, the two components are co-administered to patients having a lower than threshold TRIP12 activity in order to increase likelihood of effect.

In certain embodiments, the two components are co-administered to patients having a higher than normal, or higher than threshold TRIP12 activity in order to facilitate the administration and effect of PARP inhibitor therapy.

### Ubiquitin ligase inhibitors that can be combined with PARP inhibitors

Ubiquitin ligase inhibitors that can favourably be combined with PARP inhibitors according to the invention include, but are not limited to,
- [4-[[4-Chloro-3-(trifluoromethyl)phenyl]sulfonyl]-1-piperazinyl][4-(5-methyl-1H-pyrazol-1-yl)phenyl]methanone (A01),
- 3-(2-Methyl-5-nitroimidazol-1-yl)-N-(2,2,2-trichloro-1-phenylaminoethyl)propionamide (Apcin)
- 4-[4-[[5-(2-Fluorophenyl)-2-furanyl]methylene]-4,5-dihydro-5-oxo-3-(phenylmethyl)-1H-pyrazol-1-yl]benzoic acid (GS143)
- N-(4-Acetylphenyl)-3-(5-ethyl-2-furanyl)-2-propenamide (Heclin)
- 5-[[3-Dimethylamino)propyl]amino]-3,10-dimethylpyrimido[4,5-b]quinoline-2,4(3H,10H)-dione dihydrochloride (HLI373)
- 4-[[[(2*R*,3*S*,4*R*,5*S*)-3-(3-Chloro-2-fluorophenyl)-4-(4-chloro-2-fluorophenyl)-4-cyano-5-(2,2-dimethylpropyl)-2-pyrrolidinyl]carbonyl]amino]-3-methoxybenzoic acid (Idasanutlin)
- 2-Methyl-7-[Phenyl(phenylamino)methyl]-8-quinolinol (NSC 66811)
- (±)-4-[4,5-*Bis*(4-chlorophenyl)-2-(2-isopropoxy-4-methoxy-phenyl)-4,5-dihydroimidazole-1-carbonyl]-piperazin-2-one (Nutlin-3)
- 4-[[(4*S*,5*R*)-4,5-Bis(4-chlorophenyl)-4,5-dihydro-2-[4-methoxy-2-(-methylethoxy)phenyl]-1*H*-imidazol-1-yl]carbonyl]-2-piperazinone (Nutlin-3a)
- 4-Amino-2-(2,6-dioxo-3-piperidinyl)-1*H*-isoindole-1,3(2*H*)-dione (Pomalidomide)
- 2-(3-Pyridinylmethylene)-1*H*-Indene-1,3(2*H*)-dione (PRT-4165)
- *N*-(2,6-Dibromo-4-methoxyphenyl)-4-(2-methylimidazo[1,2-a]pyrimidin-3-yl)-2-thiazolamine (PTC 209)
- 5,5'-(2,5-Furandiyl)*bis*-2-thiophenemethanol (RITA)
- 2-[4-Bromo-2-[[4-oxo-3-(3-pyridinylmethyl)-2-thioxo-5 thiazolidinylidene]methyl]phenoxy]acetic acid (SKPin C1)
- 9*H*-Indeno[1,2-*e*][1,2,5]oxadiazolo[3,4-*b*]pyrazin-9-one (SMER 3)
- 6-Methoxy-1-(1-naphthalenyl)-9*H*-pyrido[3,4-*b*]indole (SP141)
- 3-(2-Benzothiazolyl)-6-ethyl-7-hydroxy-8-(1-piperidinylmethyl)-4H-1-benzopyran-4-one (SZL P1-41)
- *N*²-[(4-Methylphenyl)sulfonyl]-L-arginine methyl ester hydrochloride (TAME hydrochloride)
- *N*-(2,6-dioxo-3-piperidinyl)phthalimide (Thalidomide)
- (2S,4*R*)-1-((*S*)-2-(1-cyanocyclopropanecarboxamido)-3,3-dimethylbutanoyl)-4-hydroxy-*N*-(4-(4-methylthiazol-5-yl)benzyl)pyrrolidine-2-carboxamide (VH 298)
- Ethyl 3-[2-(*tert*-butylamino)-1-[*N*-(4-chlorobenzyl)formamido]-2-oxoethyl]-6-chloro-1*H-*indole-2-carboxylate (YH 239-EE)
- (2S)-2-[[(4-methylphenyl)sulfonyl]amino]-9,13-dioxo-14-phenyl-7-[[[[(2-phenylacetyl)oxy]methoxy]carbonyl]amino]-10,12-dioxa-6,8-diazatetradec-6-enoic acid,methylester (proTAME)
- 3-(4-Amino-1,3-dihydro-1-oxo-2*H*-isoindol-2-yl)-2,6-piperidinedione (Lenalidomide)
- (2*S*,4*R*)-1-((*S*)-2-(tert-butyl)-17-((*S*)-4-(4-chlorophenyl)-2,3,9-trimethyl-6*H*-thieno[3,2-*f*][1,2,4]triazolo[4,3-a][1,4]diazepin-6-yl)-4,16-dioxo-6,9,12-trioxa-3,15-diazaheptadecanoyl)- 4-hydroxy-*N*-(4-(4-methylthiazol-5-yl)benzyl)pyrrolidine-2-carboxamide (MZ 1)
- *N*-(2-(2-(2-(4-(4-((5-Chloro-4-((2-(isopropylsulfonyl)phenyl)amino)pyrimidin-2-yl)amino)phenyl)piperazin-1-yl)ethoxy)ethoxy)ethyl)-2-((2-(2,6-dioxopiperidin-3-yl)-1,3-dioxoisoindolin-4-yl)amino)acetamide (TL 12-186)
- (2*S*,4*S*)-1-((*S*)-2-(tert-butyl)-17-((*S*)-4-(4-chlorophenyl)-2,3,9-trimethyl-6H-thieno[3,2-*f*][1,2,4]triazolo[4,3-*a*][1,4]diazepin-6-yl)-4,16-dioxo-6,9,12-trioxa-3,15-diazaheptadecanoyl)- 4-hydroxy-*N*-(4-(4-methylthiazol-5-yl)benzyl)pyrrolidine-2-carboxamide (cis MZ 1)
- (6*S*)-4-(4-Chlorophenyl)-*N*-[4-[[2-[[2-(2,6-dioxo-3-piperidinyl)-2,3-dihydro-1,3-dioxo-1*H*-isoindol-4-yl]oxy]acetyl]amino]butyl]-2,3,9-trimethyl-6*H*-thieno[3,2-*f*l[1,2,4]triazolo[4,3-*a*][1,4]diazepine-6-acetamide (dBET1)
- (2*S*,4*R*)-1-(2*R*)-2-Acetamido-3-[[6-[2-[(6*S*)-4-(4-chlorophenyl)-2,3,9-trimethyl-6H-thieno[3,2-f][1,2,4]triazolo[4,3-a][1,4]diazepin-6-yl]acetamido]hexyl]thio]-3-methylbutanoyl]-4-hydroxy-*N*-[4-(4-methylthiazol-5-yl)benzyl)pyrrolidinine-2-carboxamide (AT 1)
- 2-Methyl-7-[Phenyl(phenylamino)methyl]-8-quinolinol (NSC 66811)
- 5-[[3-Dimethylamino)propyl]amino]-3,10-dimethylpyrimido[4,5-*b*]quinoline-2,4(3*H*,10*H*)-dione dihydrochloride (HLI 373)
- *N*-[(2-Chlorophenyl)methyl]-1-(2,5-dimethylphenyl)-1*H*-benzimidazole-5-carboxamide (NAB 2)
- 4-[4-[[5-(2-Fluorophenyl)-2-furanyl]methylene]-4,5-dihydro-5-oxo-3-(phenylmethyl)-1*H*-pyrazol-1-yl]benzoic acid (GS 143)
- (2*S*,4*S*)-1-((*S*)-2-(1-cyanocyclopropanecarboxamido)-3,3-dimethylbutanoyl)-4-hydroxy-*N*-(4-(4-methylthiazol-5-yl)benzyl)pyrrolidine-2-carboxamide (*cis* VH 298)
- *N*¹,*N*²⁰-bis((*S*)-1-((2*S*,4*R*)-4-Hydroxy-2-((4-(4-methylthiazol-5-yl)benzyl)carbamoyl)pyrrolidin-1-yl)-3,3-dimethyl-1-oxobutan-2-yl)-3,6,9,12,15,18-hexaoxaicosanediamide (CM 11)
- *N*¹,N²⁰-bis((*S*)-1-((2*S*,4*S*)-4-Hydroxy-2-((4-(4-methylthiazol-5-yl)benzyl)carbamoyl)pyrrolidin-1-yl)-3,3-dimethyl-1-oxobutan-2-yl)-3,6,9,12,15,18-hexaoxaicosanediamide (CMP 98)
- *N*-(2-(2-(2-(4-(4-((5-Chloro-4-((2-(isopropylsulfonyl)phenyl)amino)pyrimidin-2-yl)amino)phenyl)piperazin-1-yl)ethoxy)ethoxy)ethyl)-2-((1,3-dioxo-2-(2-oxopiperidin-3-yl)isoindolin-4-yl)amino)acetamide (TL 13-27)
- *N*¹-(3-Chloro-1*H*-indol-7-yl)-1,4-benzenedisulfonamide (Idisulam)
- 4-[[[(2*R*,3*S*,4*R*,5*S*)-3-(3-Chloro-2-fluorophenyl)-4-(4-chloro-2-fluorophenyl)-4-cyano-5-(2,2-dimethylpropyl)-2-pyrrolidinyl]carbonyl]amino]-3-methoxybenzoic acid (Indasanutlin)
- (2S,4R)-1-((2S)-2-(5-((5-((6-((2-(2,6-Dioxopiperidin-3-yl)-1,3-dioxoisoindolin-4-yl)amino)hexyl)oxy)pentyl)oxy)pentanamido)-3,3-dimethylbutanoyl)-4-hydroxy-*N*-(4-(4-methylthiazol-5-yl)benzyl)pyrrolidine-2-carboxamide (CRBN-6-5-5-VHL)
- 2-Thiophenemethanol, 5,5'-(2,5-furandiyl)bis- (RITA NSC 652287)
- Benzamide, N-[[[4-(acetylamino)phenyl]amino]thioxomethyl]-4-(1,1-dimethylethyl)-(Tenovin-1)
- (5S,8S,10aR)-N-benzhydryl-5-((S)-2-(methylamino)propanamido)-3-(3-methylbutanoyl)-6-oxo- decahydropyrrolo[1,2-a][1,5]diazocine-8-carboxamide (AT 406 (SM-406))
- 2,6-Piperidinedione, 3-(5-amino-2-methyl-4-oxo-3(4H)-quinazolinyl)- (Avadomide (CC-122))
- 2,1,3-Benzoxadiazole, 4-(4-methyl-1-piperazinyl)-7-nitro-, 3-oxide (NSC 207895)
- N1-(2-(1H-indol-3-yl)ethyl)-N4-(pyridin-4-yl)benzene-1,4-diamine (JNJ-26854165 (Serdemetan))
- 2,6-Piperidinedione, 3-[1,3-dihydro-4-[[4-(4-morpholinylmethyl)phenyl]methoxy]-1-oxo-2H-isoindol-2-yl]-, (3S)- (lberdomide(CC220))

### Nucleic acid agents that can be combined with PARP inhibitors according to the invention

A range of agents and techniques are known to the skilled person that facilitate the inhibition or abrogation of expression of a target gene in cells. These include antisense oligonucleotides that are capable of hybridizing to mRNA. Antisense oligonucleotides can be made of DNA oligonucleotides, RNA, modified DNA or nucleotide analogues such as LNA or PNA, or a mix of some or all of the above. Antisense oligonucleotides often comprise phosphothioate linkages instead of phosphate linkages to protect the open ends of the oligomer from exonuclease degradation.

Similarly, a catalogue of RNA designs exists that can enable targeting of mRNA or regulatory RNA in cells, including so-called siRNA, shRNA, sgRNA and miRNA, all of which can be employed in accordance to known techniques to target TRIP12.

Furthermore, RNA antisense agents can be delivered intracellularly by administration of DNA or RNA expression vectors, from which such RNA agents are transcribed. Non-limiting examples are DNA plasmids, adenovirus and adeno-associated virus vectors.

In certain embodiments, the nucleic acid agent is an inhibitory RNA or an antisense oligonucleotide capable of hybridizing to a TRIP12 mRNA, or a polynucleotide capable of expressing an inhibitory RNA capable of hybridizing to a TRIP12 mRNA.

### PARP inhibitors that can be combined with nucleic acid agents or ubiquitin ligase inhibitors

In certain embodiments of this aspect of the invention, the PARP inhibitor is Olaparib (Lynparza®, CAS No. 763113-22-0). In certain embodiments, the PARP inhibitor is Rucaparib (Rubraca®, CAS No. 283173-50-2). In certain embodiments, the PARP inhibitor is Talazoparib (Talzenna®, CAS No CAS No. 1207456-01-6). In certain embodiments, the PARP inhibitor is Veliparib (ABT-888; CAS No 912444-00-9). In certain embodiments, the PARP inhibitor is Niraparib (Nejula®, CAS No 1038915-60-4). In certain embodiments, the PARP inhibitor is Pamiparib (CAS No.: 1446261-44-4). In certain embodiments, the PARP inhibitor is CEP-9722 (11-methoxy-2-((4-methylpiperazin-1-yl)methyl)-4,5,6,7-tetrahydro-1 H-cyclopenta[a]pyrrolo[3,4-c]carbazole-1,3(2H)-dione; CAS#: 916574-83-9).

Similarly within the scope of the present invention is a method of treatment of a tumour disease in a patient in need thereof, comprising administering to the patient a PARP inhibitor or a combination of a PARP inhibitor and a TRIP12 inhibitor or a ubiquitin ligase inhibitor according to the above description.

### Pharmaceutical Composition and Administration

Another aspect of the invention relates to a pharmaceutical composition comprising a compound of the present invention, or a pharmaceutically acceptable salt thereof, and a pharmaceutically acceptable carrier, for use in cancer treatment of patients identified according to the invention as related herein. In further embodiments, the composition comprises at least two pharmaceutically acceptable carriers, such as those described herein.

In certain embodiments of the invention, the compound of the present invention is typically formulated into pharmaceutical dosage forms to provide an easily controllable dosage of the drug and to give the patient an elegant and easily handleable product.

The pharmaceutical composition can be formulated for oral administration, parenteral administration, or rectal administration. In addition, the pharmaceutical compositions of the present invention can be made up in a solid form (including without limitation capsules, tablets, pills, granules, powders or suppositories), or in a liquid form (including without limitation solutions, suspensions or emulsions).

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of active ingredient(s) as known in the art for a subject of about 50-70 kg, for the specific compound. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The invention is further illustrated by the following examples and figures, from which further embodiments and advantages can be drawn. These examples are meant to illustrate the invention but not to limit its scope.

### Description of the Figures

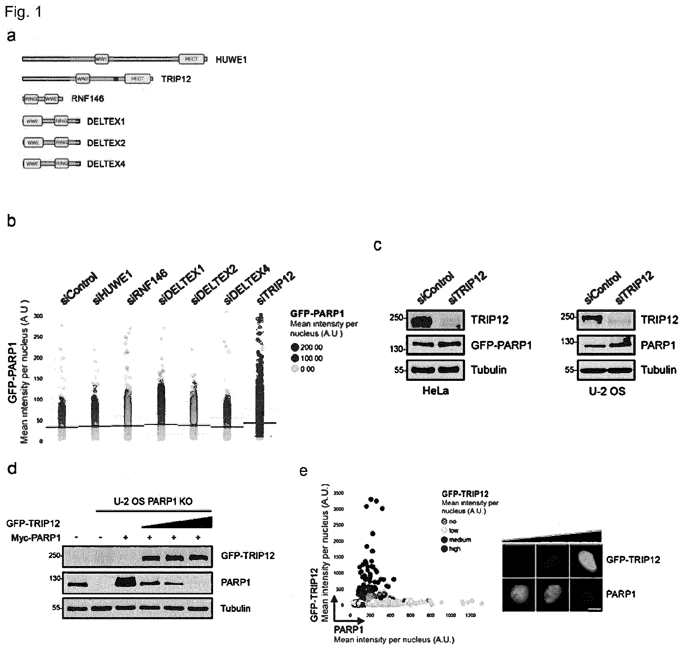

- Fig. 1: **(a)** Schematic representation of WWE domain-containing ubiquitin E3 ligases. HECT, homologous to the E6-AP carboxyl terminus; RING, really interesting new gene. **(b)** HeLa Kyoto PARP1-LAP cells expressing GFP-PARP1 from its natural promoterwere transfected with negative control siRNA or with siRNAs against WWE- containing ubiquitin E3 ligases. Nuclear GFP-PARP1 levels were analyzed 72h after siRNA transfection by high-content microscopy. Pooled results from two independent siRNAs against each target protein are shown. Mean (solid line) and standard deviation from the mean (dashed lines) are indicated. **(c)** Western blot analysis of GFP-PARP1 levels in HeLa Kyoto PARP1-LAP cells transfected with negative control siRNA or siRNA against TRIP12. **(d)** Western blot analysis of endogenous PARP1 levels in U-2 OS cells transfected with the indicated siRNAs. **(e)** Naive U-2 OS cells (first lane) and U-2 OS PARP1 knockout (KO) cells transfected with Myc- PARP1 and increasing amounts of GFP-TRIP12 (0, 1, 2, 4 µg of plasmid, respectively) were analyzed for PARP1 levels by Western blot. **(f)** U-2 OS transfected with GFP-TRIP12 were analyzed by high-content microscopy for GFP-TRIP12 and endogenous PARP1 levels. Nuclear GFP-TRIP12 and PARP1 intensities per cell are plotted, color-coded by GFP-TRIP12 expression. Representative single cell images are shown to the right. Scale bar, 10 µm.

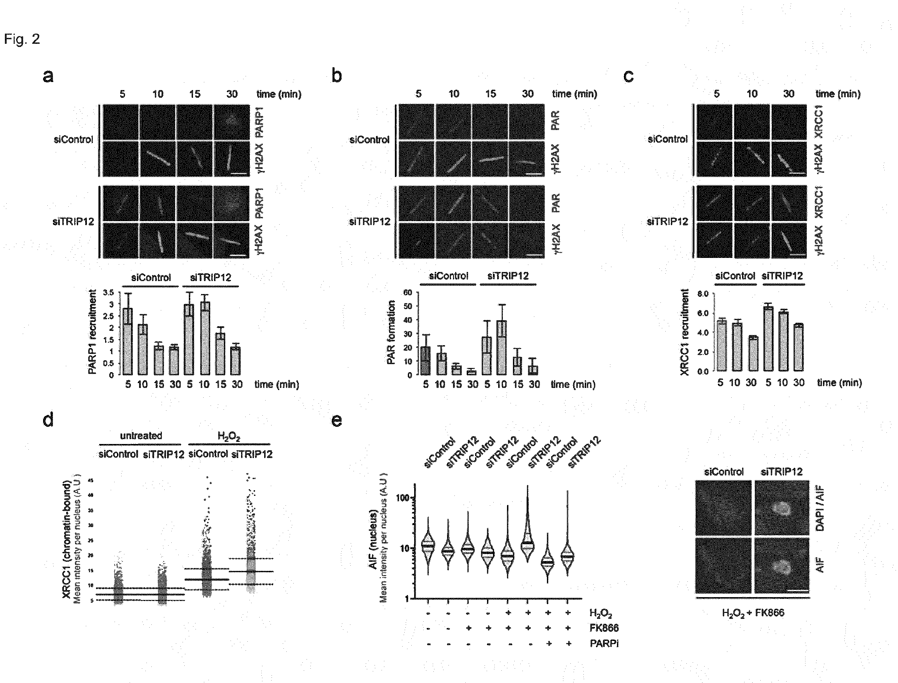

- Fig. 2: **(a)** U-2 OS cells were transfected with the indicated siRNAs and localized DNA damage was induced by laser micro-irradiation. Cells were allowed to recover for the indicated time periods and stained for γH2AX as DNA damage marker and PARP1. Representative single cell images are shown and quantification of PARP1 recruitment to sites of DNA damage is depicted below as mean ± SD. **(b)** U-2 OS cells were treated as in (a) and stained for γH2AX and PAR. Representative single cell images are shown and quantification of PAR formation at sites of DNA damage is depicted below as mean ± SD. **(c)** U-2 OS cells were treated as in (a) and stained for γH2AX and XRCC1. Representative single cell images are shown and quantification of XRCC1 recruitment to sites of DNA damage is depicted below as mean ± SD. **(d)** U-2 OS cells were transfected with siRNAs and exposed to 0.1 mM H2O2 for 1h as indicated. Cells were pre-extracted, fixed and chromatin-associated XRCC1 levels were analyzed by high-content microscopy. Mean (solid line) and standard deviation from the mean (dashed lines) are indicated. **(e)** U-2 OS cells were transfected with siRNAs and treated as indicated (FK866: 10 µM, 24h; PARPi Olaparib: 10 µM, 24h; H2O2: 20 µM, 12h) to induce PARthanatos-associated AIF release from mitochondria. High-content imaging-derived violin plot show nuclear AIF intensities. Median (solid line) and quartiles (dashed lines) are indicated. Representative single cell images of AIF translocation to the nucleus upon combined H2O2 and FK688 treatment are shown to the right. Scale bars, 10 µm.

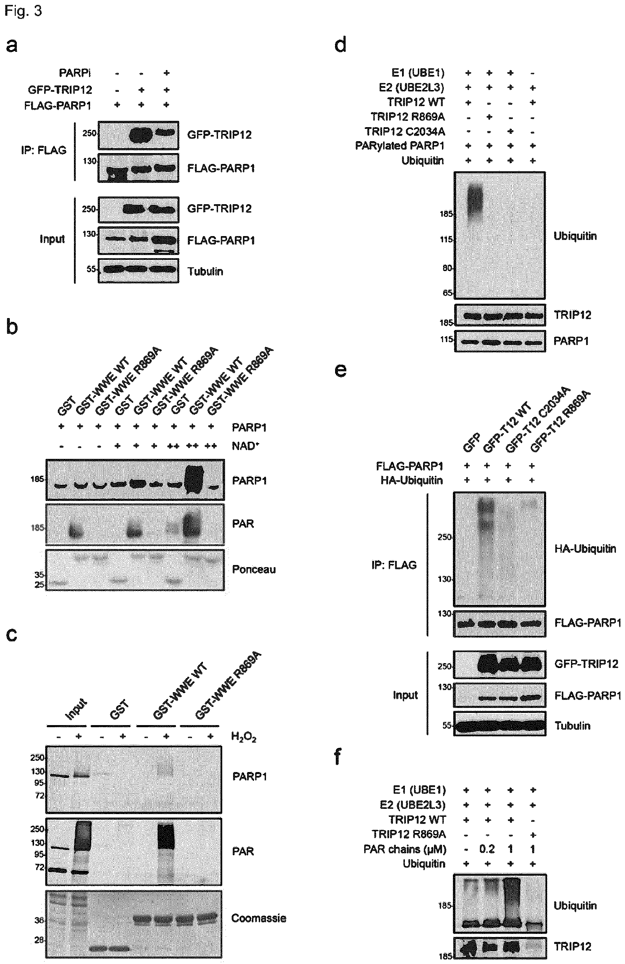

- Fig. 3: **(a)** HEK293T cells were transfected with the indicated plasmids for co-immunoprecipitation (co-IP) experiments, with or without prior PARPi treatment (Olaparib: 10 µM, 1h). FLAG-PARP1 was immunoprecipitated and the interaction with GFP-PARP1 was analyzed by Western blot. **(b)** *In vitro* interaction assay of purified GST, GST-TRIP12-WWE wild-type (WT) and GST- TRIP12-WWE R869A mutant with autoPARylated PARP1. Recombinant purified PARP1 was incubated with a low (20 µM, +) or high (200 µM, ++) concentration of NAD+ for 15 minutes at 30°C to induce PARP1 autoPARylation prior to the GST interaction assay. PARP1 and PAR binding to the purified GST-fusion proteins was assessed by Western blot. **(c)** *Ex vivo* interaction assay of purified GST, GST- TRIP12-WWE wild-type (WT) and GST-TRIP12-WWE R869A mutant with whole cell lysates from HeLa cells, which were exposed to 0.1 mM H2O2 for 10 minutes prior to cell lysis to induce PAR formation. PARG was depleted from these cells by siRNA to avoid the PARG-mediated rapid degradation of PAR. PARP1 and PAR binding to the purified GST-fusion proteins was assessed by Western blot. **(d)** *In vitro* pubiquitylation assay with purified E1 (UBE1), E2 (UBE2L3), E3 (FLAG-TRIP12 wild-type (WT), WWE mutant (R869A), or HECT mutant (C2034A) purified from HEK293T cells) and ubiquitin, using autoPARylated PARP1 as a target protein. PARP1 ubiquitylation was assessed by Western blot. **(e)** *In vivo* ubiquitylation assay in HEK293T cells expressing FLAG-PARP1 and GFP-TRIP12 wild-type (WT), WWE mutant (R869A), or HECT mutant (C2034A) together with HA-ubiquitin. FLAG-PARP1 was immunoprecipitated and PARP1 ubiquitylation was assessed by Western blot. **(f)** *In vitro* ubiquitylation assay to monitor TRIP12 activity in absence or presence of purified PAR chains. FLAG-TRIP12 wild-type (WT) or the PAR- binding-deficient WWE mutant (R869A) were purified from HEK293T cells and incubated with E1 (UBE1), E2 (UBE2L3) and ubiquitin with or without different amounts of purified PAR chains as indicated. Auto-ubiquitylation of TRIP12 was assessed by Western blot.

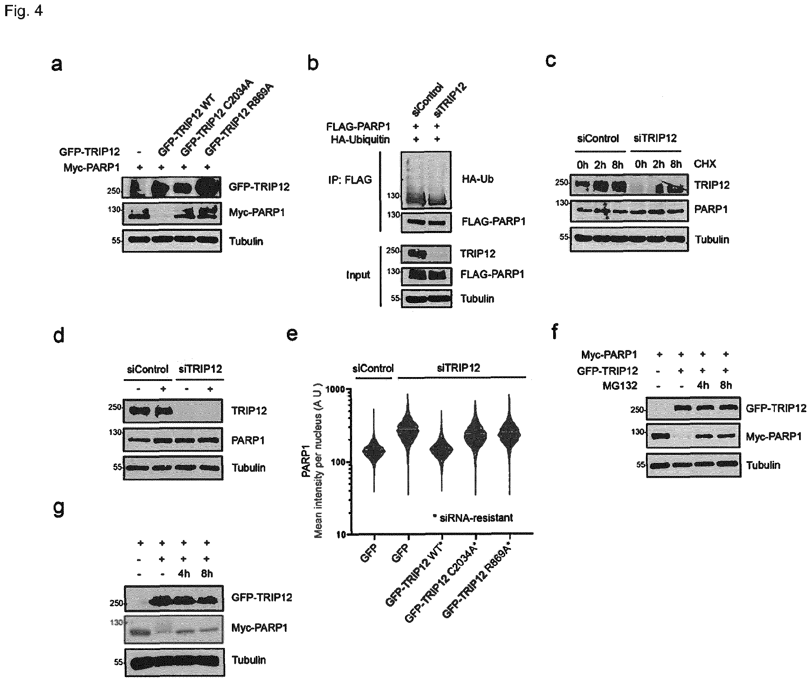

- Fig. 4: **(a)** U-2 OS PARP1 KO cells were transfected with the indicated plasmids and the effect of GFP-TRIP12 expression on Myc-PARP1 levels was assessed by Western blot. **(b)** U-2 OS cell were transfected with control siRNA or siRNA against TRIP12 for 48h, followed by the indicated plasmid transfections for 24h. FLAG-PARP1 was immunoprecipitated and its ubiquitylation was assessed by Western blot. **(c)** U-2 OS were transfected with control siRNA or siRNA against TRIP12 and exposed to cycloheximide (CHX, 50 µM) for the indicated time periods. TRIP12 and PARP1 levels were assessed by Western blot. **(d)** U-2 OS were transfected with control siRNA or siRNA against TRIP12 and exposed to PARPi (PJ- 34, 10 µM) for 16h as indicated. TRIP12 and PARP1 levels were assessed by Western blot. **(e)** U-2 OS cell were transfected with control siRNA or siRNA against TRIP12 for 48h, followed by the indicated plasmid transfections for 24h. All TRIP12 plasmids were rendered siRNA-resistant by introducing multiple silent mutations. High-content imaging-derived violin plot show nuclear PARP1 intensities. Median (solid line) and quartiles (dashed lines) are indicated. **(f)** U-2 OS PARP1 KO cells were transfected with plasmids for Myc-PARP1 and GFP-TRIP12 expression and exposed to the proteasome inhibitor MG132 (10 µM) as indicated. PARP1 levels were assessed by Western blot. **(g)** U-2 OS PARP1 KO cells were transfected as in (f) and exposed to PARPi (Olaparib, 10 µM) as indicated. PARP1 levels were assessed by Western blot.

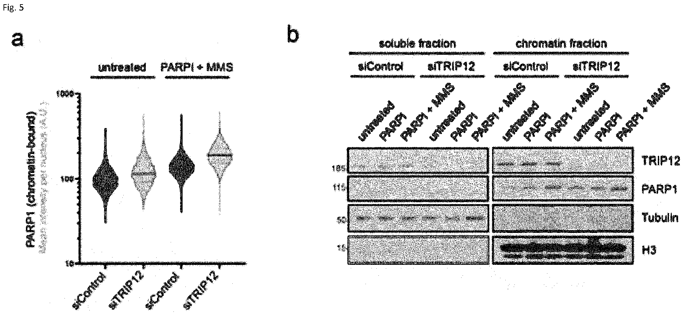

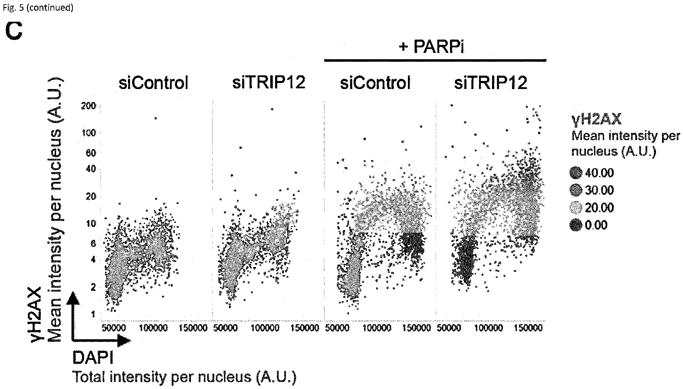

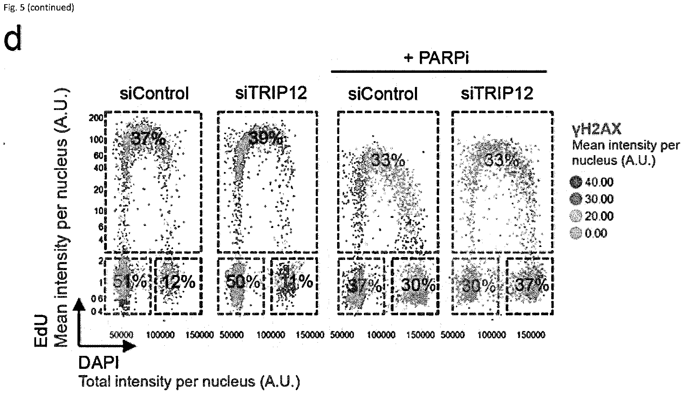

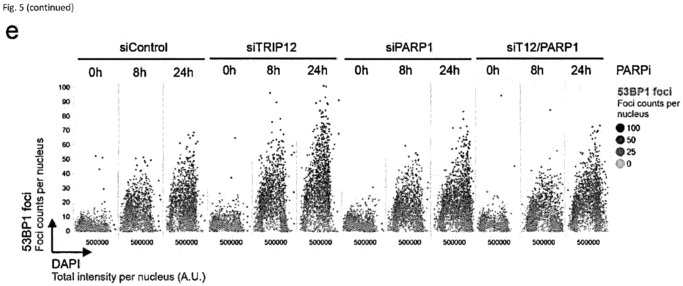

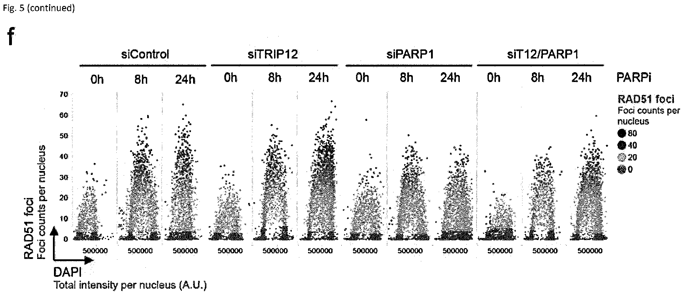

- Fig. 5: **(a)** U-2 OS cells transfected with control siRNA or siRNA against TRIP12 and exposed to MMS (0.01%) and PARPi (Olaparib 10 µM) for 1h as indicated. Cells were pre-extracted and chromatin-bound PARP1 was analyzed by high-content microscopy. Violon plots with median (solid line) and quartiles (dashed lines) are shown. **(b)** U-2 OS cells transfected with control siRNA or siRNA against TRIP12 were treated with MMS (0.01%) and PARPi (Olaparib 10 µM) for 2h as indicated. Cells were then subjected to chromatin fractionation to separate the soluble from the chromatin-bound fraction. Chromatin-bound PARP1 was analyzed by Western blot. **(c)** HeLa Kyoto PARP1-LAP cells were transfected with control siRNA or siRNA against TRIP12 and exposed to PARPi (Olaparib 10 µM) for 16h. QIBC- derived cell cycle resolved γH2AX profiles are shown. **(d)** QIBC-derived EdU profiles are shown and percentages of cells in G1, S, and G2 are provided for the same cells analyzed in (c). **(e)** U-2 OS cells were transfected with siRNAs and exposed to PARPi (Olaparib 10 µM) as indicated. QIBC-derived cell cycle resolved profiles of nuclear 53BP1 foci are shown. **(f)** U-2 OS cells were treated as in (e) and QIBC-derived cell cycle resolved profiles of nuclear RAD51 foci are shown.

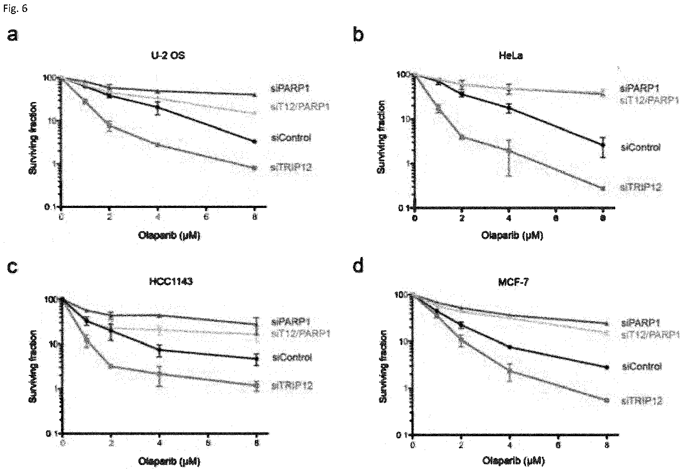

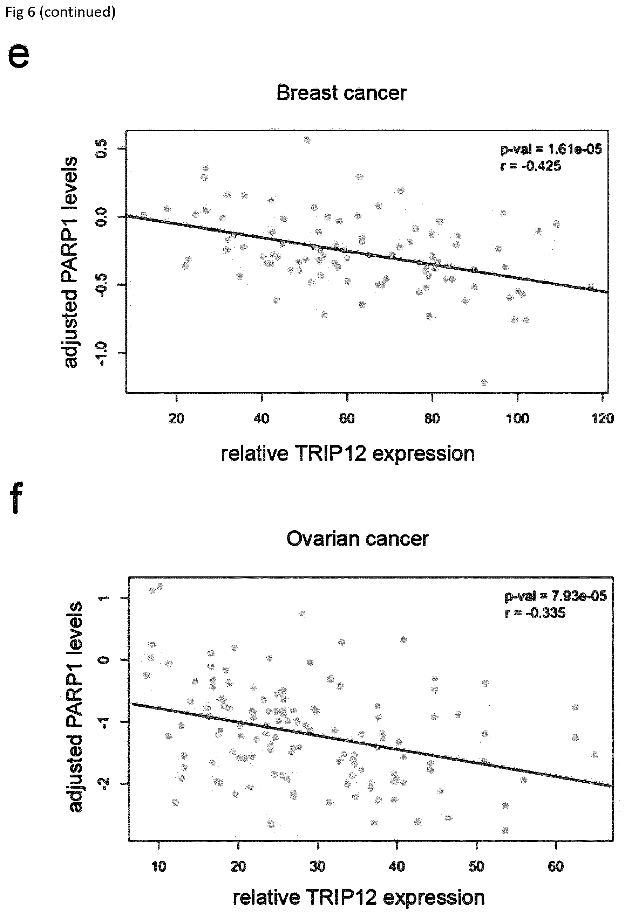

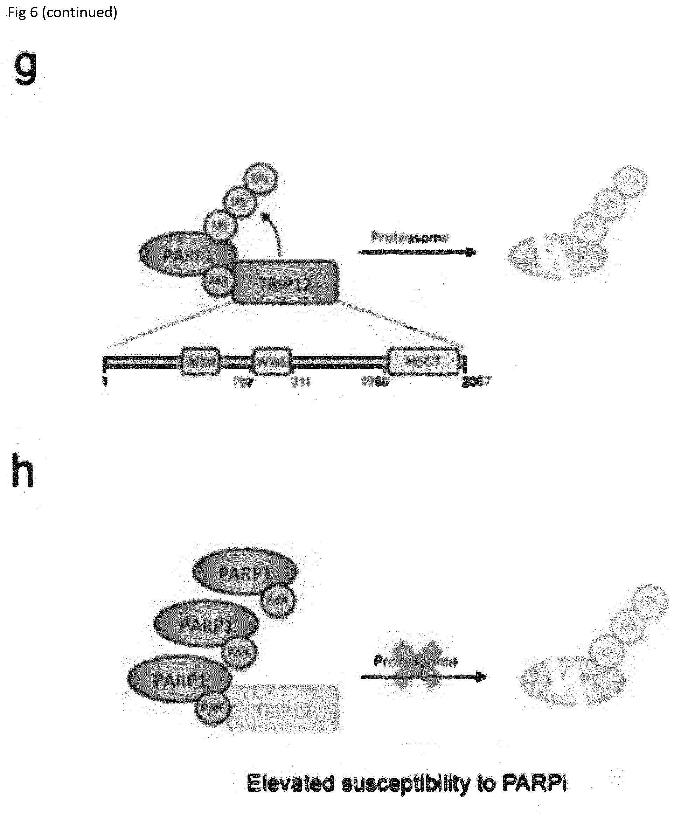

- Fig. 6: **(a)** U-2 OS cells were transfected with siRNAs and exposed to PARPi Olaparib as indicated for clonogenic survival assays. Relative clonogenic survival in response to increasing doses of PARPi Olaparib is shown. **(b)** HeLa Kyoto PARP1-LAP cells were treated as in (a) and relative clonogenic survival in response to increasing doses of PARPi Olaparib is shown. **(c)** HCC1143 cells were treated as in (a) and relative clonogenic survival in response to increasing doses of PARPi Olaparib is shown. **(d)** MCF-7 cells were treated as in (a) and relative clonogenic survival in response to increasing doses of PARPi Olaparib is shown. Means ± SD are depicted. **(e)** Scatter plot of adjusted PARP1 protein levels against the relative TRIP12 RNA expression in human breast cancer samples. Upper right shows p-value and r in Pearson's product-moment correlation test. **(f)** Scatter plot of adjusted PARP1 protein levels against the relative TRIP12 RNA expression in human ovarian cancer samples. Upper right shows p- value and r in Pearson's product-moment correlation test. **(g)** Model for PAR-targeted ubiquitylation of PARP1 by TRIP12 and the ensuing proteasomal degradation of PARP1. **(h)** When TRIP12 function is impaired, due to mutation or down-regulation, PARP1 accumulates to supra-physiological levels, which renders cancer cells hyper- sensitive to PARPi.

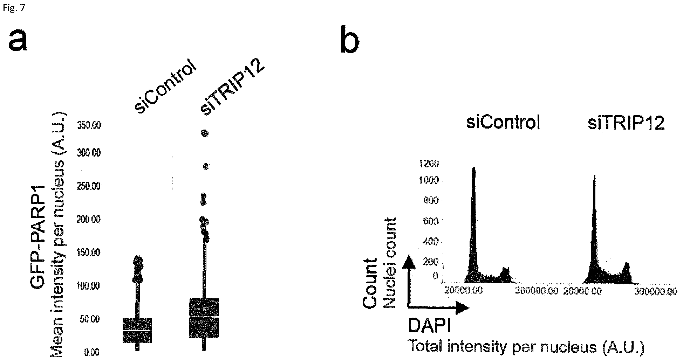

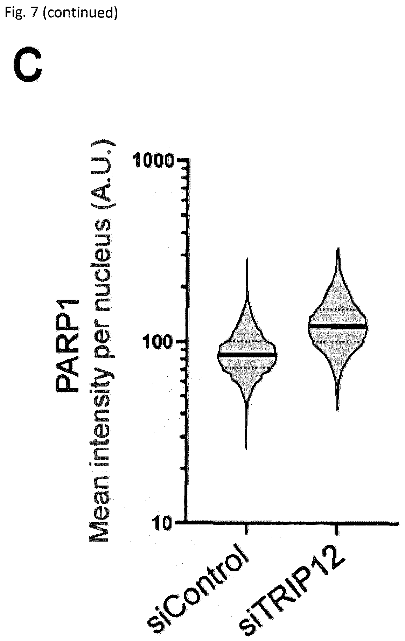

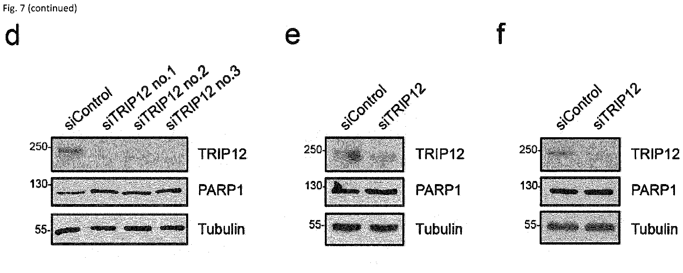

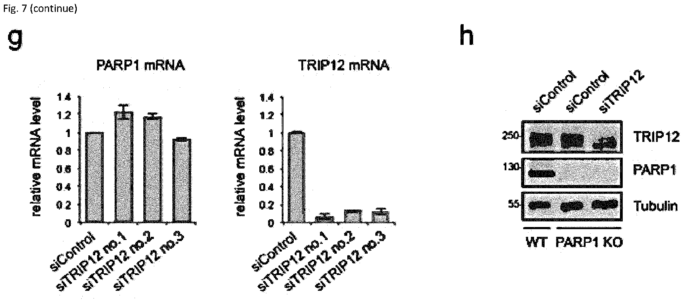

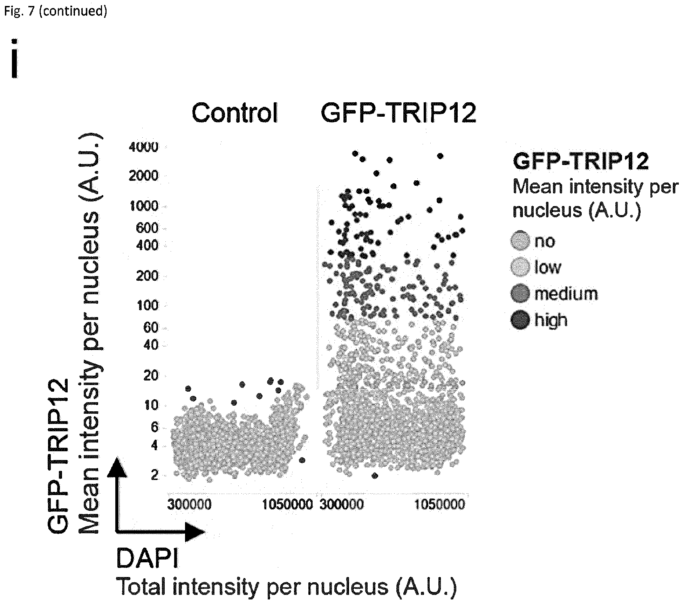

- Fig. 7: **(a)** HeLa Kyoto LAP-PARP1 cells were transfected with control siRNA or siRNA against TRIP12 and nuclear GFP-PARP1 levels were analyzed by high-content microscopy. Box plots with white lines indicating the median are shown. **(b)** QIBC-derived cell cycle profiles based on the total DAPI intensity per nucleus of the samples shown in (a). **(c)** U-2 OS cells were transfected with control siRNA or siRNA against TRIP12 and nuclear PARP1 levels were analyzed by high-content microscopy. Violin plots with median (solid line) and quartiles (dashed lines) indicated are shown. **(d)** U-2 OS cells were transfected with three different siRNAs against TRIP12 (no.1, no.2, no.3) and TRIP12 and PARP1 levels were analyzed by Western blot. **(e)** RPE-1 cells were transfected with control siRNA or siRNA against TRIP12 and TRIP12 and PARP1 levels were analyzed by Western blot. **(f)** HCC1143 cells were transfected with control siRNA or siRNA against TRIP12 and TRIP12 and PARP1 levels were analyzed by Western blot. **(g)** U-2 OS cells were transfected with three different siRNAs against TRIP12 (no.1, no.2, no.3) and PARP1 and TRIP12 mRNA levels were analyzed by RT-qPCR. Relative mRNA levels are depicted as means ± SD. **(h)** U-2 OS and U-2 OS PARP1 KO cells were transfected with the indicated siRNAs and TRIP12 and PARP1 levels were analyzed by Western blot. **(i)** QIBC-derived single cell scatter plot depicting nuclear GFP levels of the samples analyzed in Figure 1g.

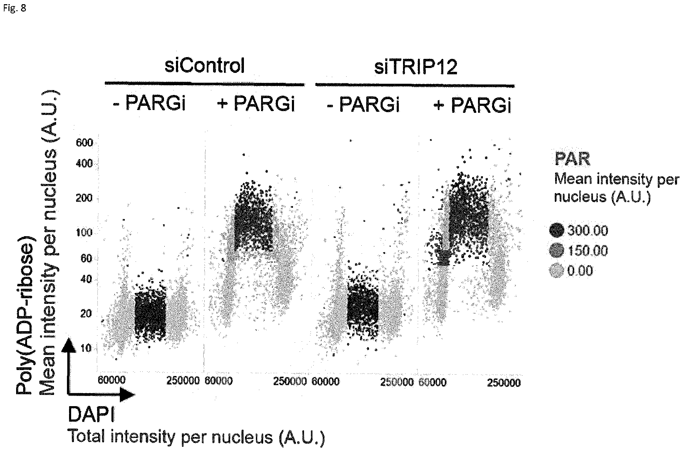

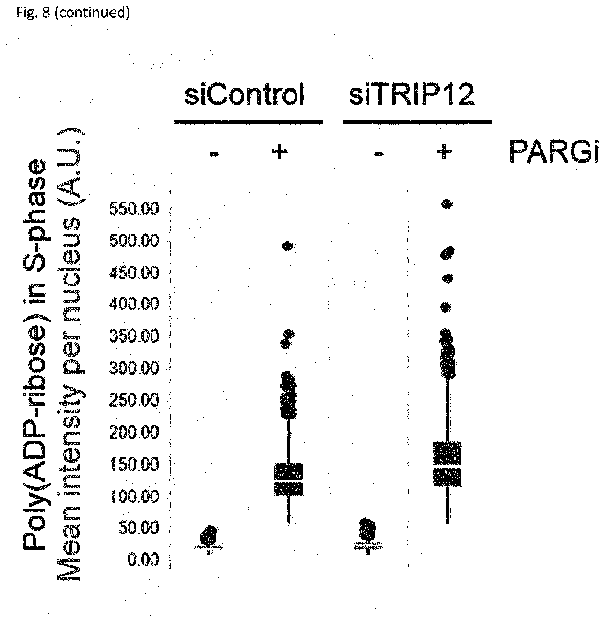

- Fig. 8: U-2 OS cells were transfected with control siRNA or siRNA against TRIP12 and exposed to PARGi (10 µM) for 1h as indicated. QIBC-derived cell cycle resolved scatter plots depict nuclear PAR levels. Box plots on the right represent PAR levels in the selected sub-populations of cells in S-phase, with white lines representing the median.

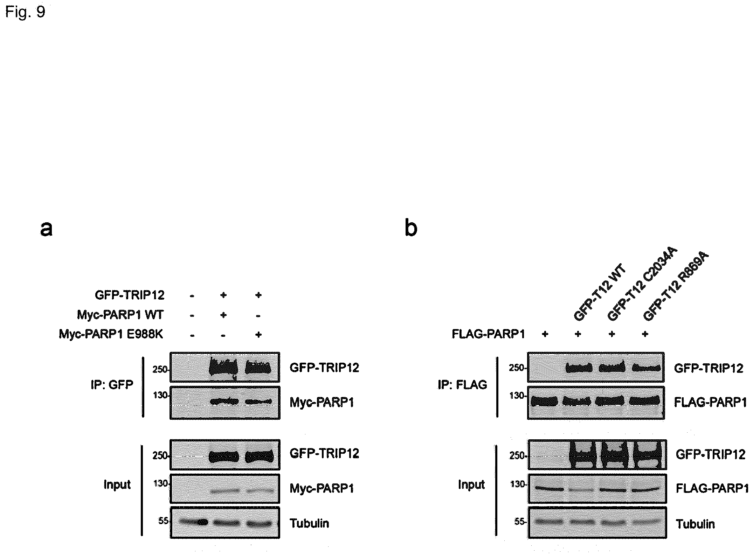

- Fig. 9: **(a)** HEK293T cells were transfected with the indicated plasmids, GFP-TRIP12 was immunoprecipitated and the interaction with Myc-PARP1 (WT or catalytically impaired E988K) was analyzed by Western blot. **(b)** HEK293T cells were transfected with the indicated plasmids, FLAG-PARP1 was immunoprecipitated and the interaction with GFP-TRIP12 (WT, HECT mutant C2034A, or WWE mutant R869A) was analyzed by Western blot.

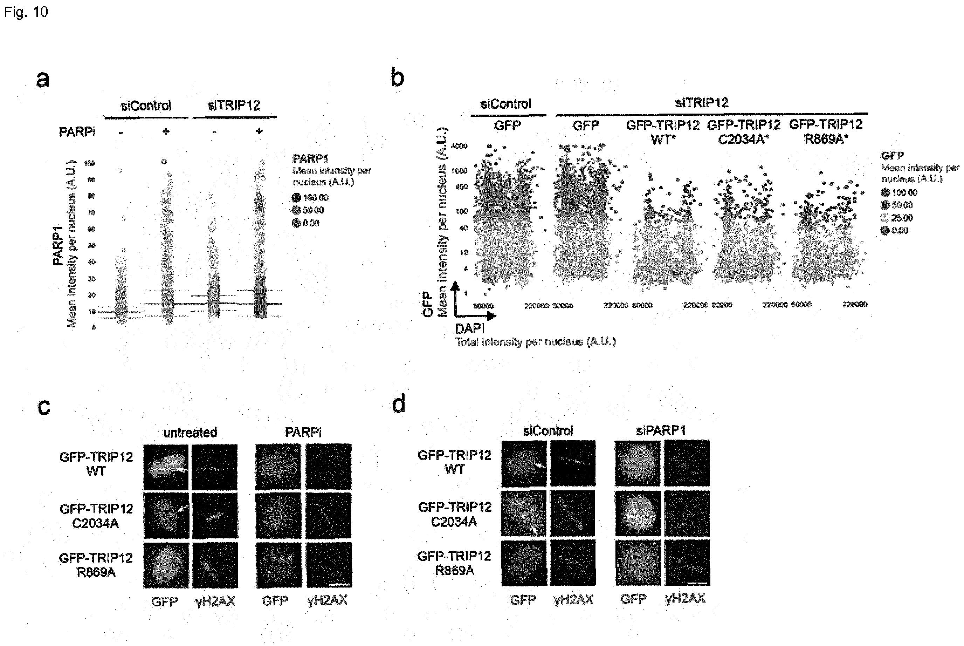

- Fig. 10: **(a)** U-2 OS cells were transfected with control siRNA or siRNA against TRIP12 and exposed to PARP1 (PJ-34, 10 µM) for 16h as indicated. Nuclear PARP1 levels were analyzed by high-content microscopy. Mean (solid line) and standard deviation from the mean (dashed lines) are indicated. **(b)** QIBC-derived GFP expression levels of the samples analyzed in Figure 4e. **(c)** U-2 OS cells were transfected with the indicated plasmids to express GFP-TRIP12 (WT, HECT mutant C2034A, or WWE mutant R869A) and subjected to laser micro-irradiation in absence or presence of PARPi (Olaparib, 10 µM). Cells were allowed to recover for 5 minutes after laser damage, were then fixed and stained for γH2AX, and GFP-TRIP12 recruitment to sites of DNA damage was analyzed. **(d)** As in (c) with U-2 OS cells either transfected with control siRNA or siRNA against PARP1. Scale bar, 10 µm.

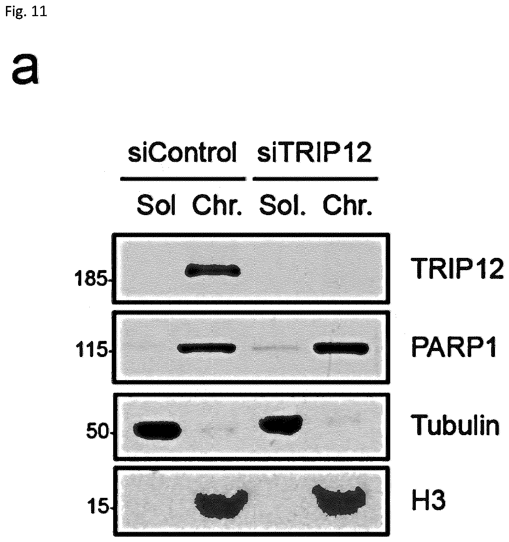

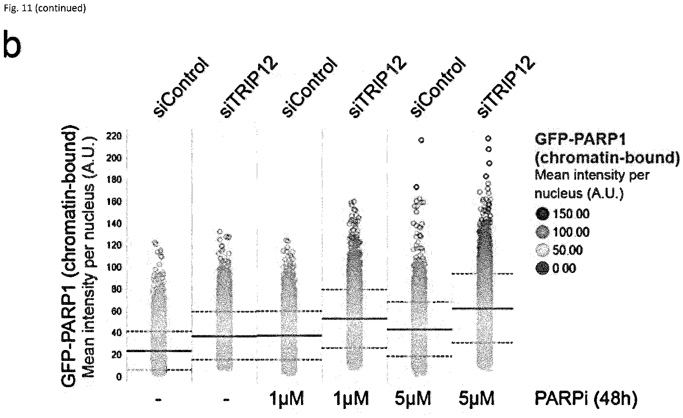

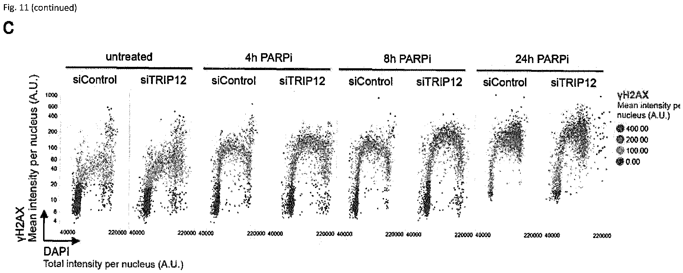

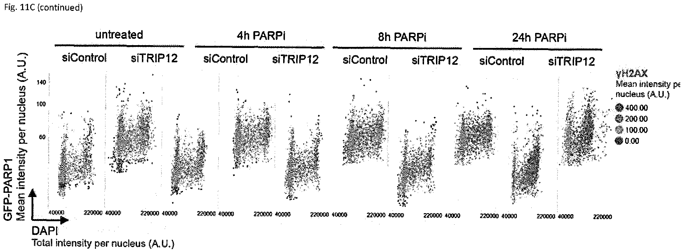

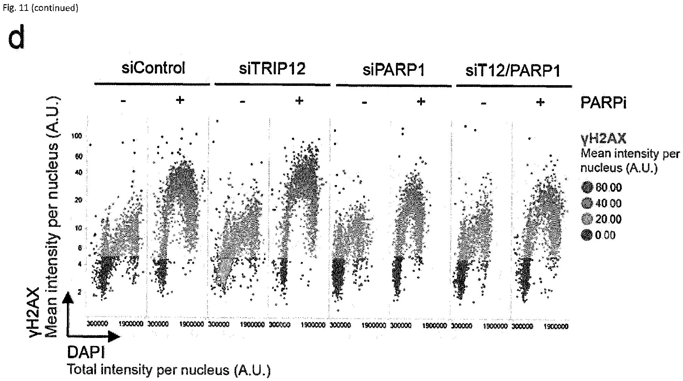

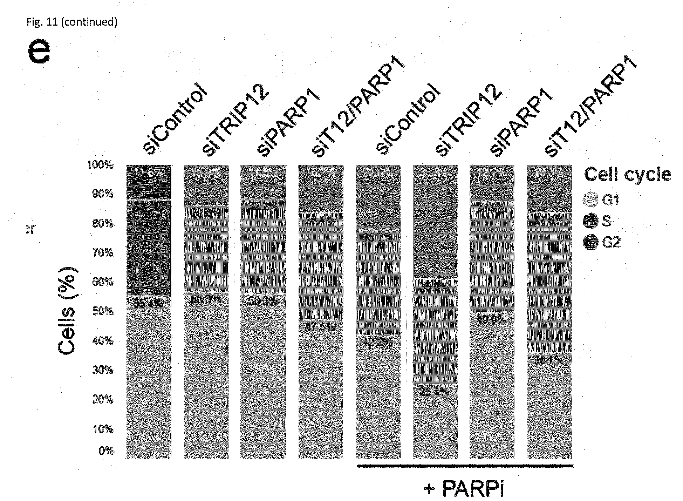

- Fig. 11: **(a)** Chromatin fractionation of U-2 OS cells transfected with the indicated siRNAs. TRIP12 and PARP1 chromatin association was analyzed by Western blot. **(b)** HeLa Kyoto PARP1-LAP cells were transfected with control siRNA or siRNA against TRIP12 and exposed to PARPi as indicated. Cells were pre- extracted and chromatin-bound GFP-PARP1 was analyzed by high-content microscopy. Mean (solid line) and standard deviation from the mean (dashed lines) are indicated. **(c)** HeLa Kyoto PARP1-LAP cells were transfected with siRNA as indicated and exposed to PARPi (Olaparib, 10 µM) for increasing time periods. QIBC-derived cell cycle resolved γH2AX and GFP-PARP1 profiles are shown. **(d)** HeLa Kyoto PARP1-LAP cells were transfected with siRNA and exposed to PARPi (Olaparib, 10 µM) for 16h as indicated. QIBC-derived cell cycle resolved γH2AX profiles are shown. **(e)** QIBC-derived cell cycle distribution based on total DAPI intensity and EdU incorporation of cells treated as in (d).

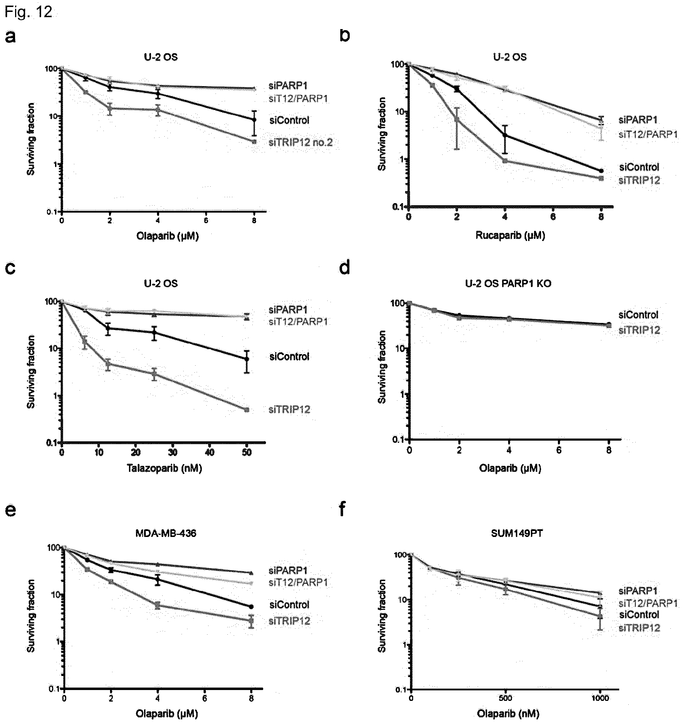

- Fig. 12: **(a)** U-2 OS cells were transfected with siRNAs and exposed to PARPi Olaparib as indicated to measure clonogenic survival. A different siRNA targeting TRIP12 (no. 2, s17809) was used compared to the siRNA used in Figure 6a. Relative clonogenic survival in response to increasing doses of PARPi Olaparib is shown. **(b)** U-2 OS cells were transfected with siRNAs and exposed to PARPi Rucaparib as indicated to measure clonogenic survival. Relative clonogenic survival in response to increasing doses of PARPi Rucaparib is shown. **(c)** U-2 OS cells were transfected with siRNAs and exposed to PARPi Talazoparib as indicated to measure clonogenic survival. Relative clonogenic survival in response to increasing doses of PARPi Talazoparib is shown. **(d)** U-2 OS PARP1 KO cells were transfected with siRNAs and exposed to PARPi Olaparib as indicated to measure clonogenic survival. Relative clonogenic survival in response to increasing doses of PARPi Olaparib is shown. **(e)** MDA-MB-436 cells, with a 5396 + 1G>A mutation in the splice donor site of *BRCA1* exon 20 and allelic *BRCA1* loss, were transfected with siRNAs and exposed to PARPi Olaparib as indicated to measure clonogenic survival. Relative clonogenic survival in response to increasing doses of PARPi Olaparib is shown. **(f)** SUM149PT cells, with a *BRCA1* 2288delT mutation and allelic *BRCA1* loss, were treated as in (e) and relative clonogenic survival in response to increasing doses of PARPi Olaparib is shown. Means ± SD are depicted.

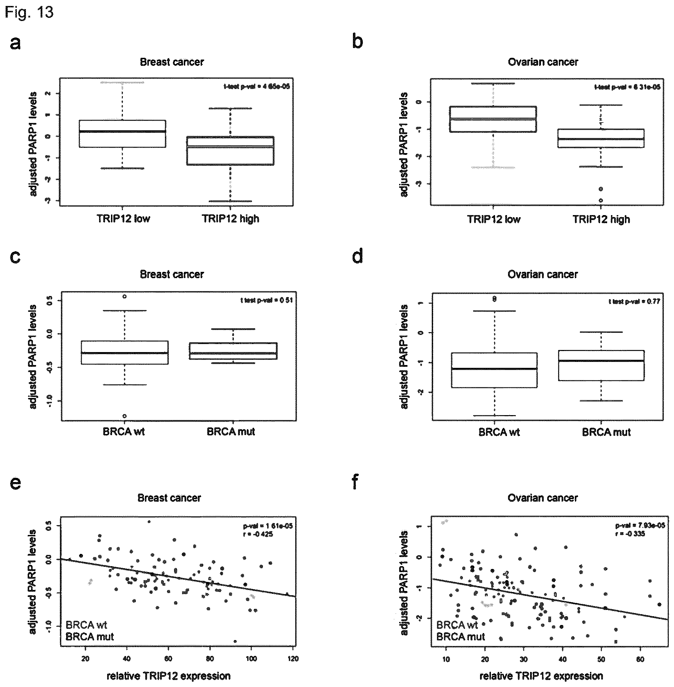

- Fig. 13: **(a)** Student t test on adjusted PARP1 protein level between sample groups equally divided based on TRIP12 RNA expression in breast cancer. **(b)** Student t test on adjusted PARP1 protein level between sample groups equally divided based on TRIP12 RNA expression in ovarian cancer. **(c)** Student t test on adjusted PARP1 protein level between BRCA wild-type and mutant samples in breast cancer. **(d)** Student t test on adjusted PARP1 protein level between BRCA wild-type and mutant samples in ovarian cancer. **(e)** Scatter plot of adjusted PARP1 protein levels against the relative TRIP12 RNA expression in breast cancer, with BRCA mutant samples highlighted. **(f)** As in (e) for the ovarian cancer samples.

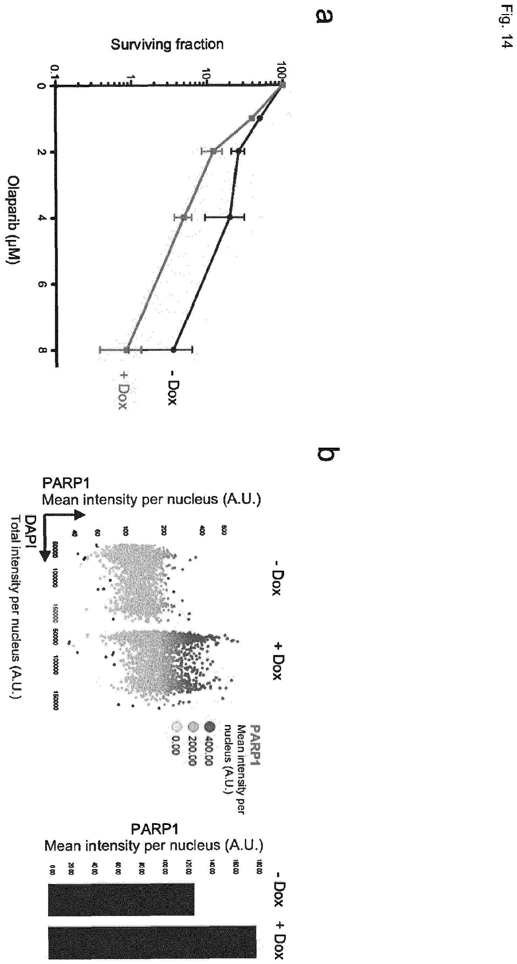

- Fig. 14: **(a)** U-2 OS cells were modified to express PARP1 in a doxycyclin (Dox) inducible manner. Cells without (- Dox) and with (+ Dox) induction of PARP1 were exposed to increasing doses of the PARP inhibitor Olaparib and clonogenic survival was assessed. **(b)** Quantitative image-based cytometry showing the degree of PARP1 induction by addition of doxycyclin. Accordingly, a 50% increase in PARP1 levels is responsible for the elevated susceptibility to Olaparib in **(a).**

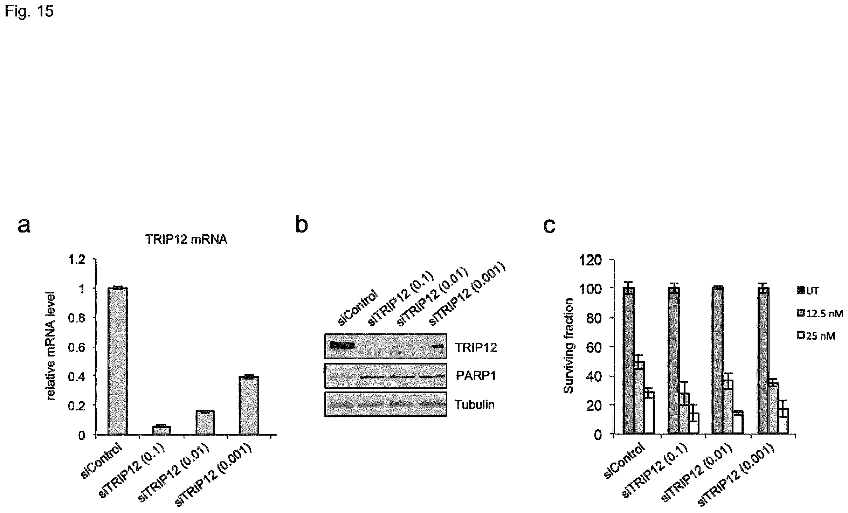

- Fig. 15: **(a)** TRIP12 was gradually depleted from U-2 OS cells by increasing doses of siRNA. TRIP12 expression was monitored by qPCR. **(b)** TRIP12 was gradually depleted from U-2 OS cells by increasing doses of siRNA. TRIP12 and PARP1 protein levels were monitored by Western blot. **(c)** U-2 OS cells were treated with increasing doses of siRNA against TRIP12, exposed to the PARP inhibitor Talazoparib at two different concentrations (12.5 nM and 25 nM), and clonogenic survival was assessed.

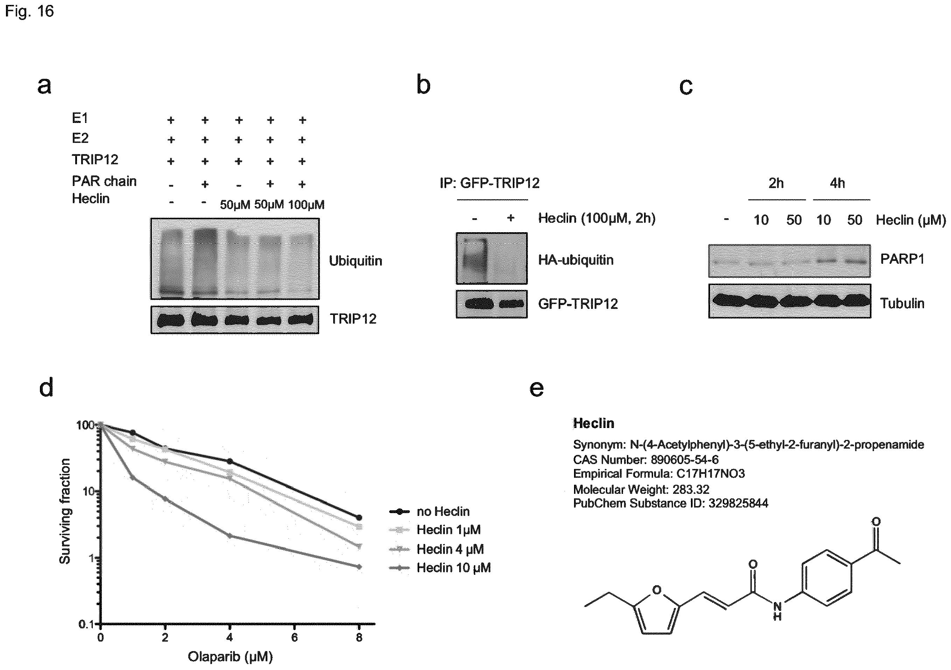

- Fig. 16: **(a)** In vitro ubiquitin ligase activity assay to measure TRIP12 activity in absence and presence of PAR chains and in absence and presence of the HECT ubiquitin ligase inhibitor Heclin. **(b)** In vivo ubiquitin ligase activity assay to measure TRIP12 activity in cells in absence and presence of the HECT ubiquitin ligase inhibitor Heclin. GFP-TRIP12 was over-expressed together with HA-ubiquitin, GFP-TRIP12 was immunoprecipitated and probed for HA-ubiquitin. **(c)** Heclin stabilizes PARP1 in U-2 OS cancer cells in a time- and dose-dependent manner. **(d)** Heclin synergizes with the PARP inhibitor Olaparib to kill cancer cells as measured by clonogenic survival. **(e)** Chemical structure of Heclin.

### Examples

### Example 1: TRIP12 counteracts PARP1 abundance

To identify novel regulators of PARP1 and PARPi sensitivity, the inventors focused on putative PAR-binding components of the ubiquitin-proteasome system. In human cells, only six ubiquitin E3 enzymes are known to combine within their structure a high affinity PAR-binding domain of the WWE type with a ubiquitin E3 ligase domain of either the HECT or RING type (Fig. 1a). Sequence alignment of the WWE domains confirms the presence of the eponymous tryptophan and glutamate residues in all six proteins. Using a previously established high-content cell imaging system to assess multiple parameters of PARPi sensitivity and resistance (Michelena, J. et al. Nature communications 9, 2678, 2018), the inventors followed an RNAi screening approach with two independent siRNAs against each target, measuring nuclear PARP1 intensities in transgenic HeLa Kyoto cells stably expressing EGFP-tagged PARP1 from its natural promoter (Aleksandrov, R. et al. Molecular cell 69, 1046-1061, e1045, 2018) as readout. This mini- screen revealed elevated PARP1 levels particularly upon down-regulation of the HECT domain ubiquitin E3 ligase TRIP12 (Fig. 1b). TRIP12, a primarily nuclear protein of around 200 kDa, which is also known as ULF, is altered in around 4% of cancer patients, with a somatic mutation frequency of 2.8% (for comparison, BRCA1 is altered in around 3% of cancer patients, with a somatic mutation frequency of 2.5% according to TCGA PanCancer Atlas data on cBioPortal). In support of the screening results, individual validation experiments by both high-content microscopy and Western blot confirmed increased PARP1 levels upon TRIP12 depletion (Fig. 1c and 7a). Cell proliferation and the cell cycle remained largely unaffected under these conditions (Fig. 7b). Similar results were obtained in U-2 OS cells treated with three independent siRNAs against TRIP12 (Fig. 1d and 7b, c), in hTERT-immortalized RPE-1 cells (Fig. 7d), and in the triple-negative breast cancer cell line HCC1143 (Fig. 7e). PARP1 mRNA levels were only very mildly affected by TRIP12 loss (Fig. 7f), suggesting that TRIP12 regulates PARP1 mainly at the protein rather than the gene expression level. In line with the knockdown experiments, over-expression of GFP-TRIP12 reduced ectopically expressed PARP1 in PARP1 knockout cells (Fig. 1f and 7h), and GFP-TRIP12 expression was inversely correlated with nuclear abundance of endogenous PARP1 (Fig. 1f and 7i). Collectively, these results reveal that nuclear PARP1 levels are negatively regulated by TRIP12.

### Example 2: TRIP12 limits PARylation and affects outcomes of PAR signaling

Consistent with elevated PARP1 after TRIP12 depletion, basal PAR formation, exposed by short-term chemical inhibition of the PAR-degrading enzyme PARG in otherwise unchallenged conditions as performed previously (Hanzlikova, H. et al. Molecular cell 71, 319, 2018), was increased (Fig. 8). Moreover, PARP1 accumulation at sites of DNA damage induced by laser micro- irradiation was more sustained (Fig. 2a), and localized PAR formation at the laser stripe was more pronounced in absence of TRIP12 (Fig. 2b). Consistent with supra- physiological PAR formation, recruitment of the PAR-binding protein XRCC1 to laser damage was enhanced upon TRIP12 depletion (Fig. 2c). Similarly, XRCC1 chromatin association in response to H2O2-induced oxidative DNA damage was elevated in TRIP12-depleted cells (Fig. 2d). Collectively, these findings suggest that TRIP12 constrains both basal and DNA damage-induced PAR formation.

As hyper-activation of PARP1 under severe conditions of genotoxic stress can lead to PARthanatos, the inventors aimed to test whether loss of TRIP12 would sensitize cells to PARP1-dependent cell death. Using AIF (apoptosis-inducing factor) translocation from the mitochondria to the nucleus as hallmark of PARthanatos upon exposure to oxidative damage in conjunction with inhibition of the NAD+ salvage pathway, elevated nuclear AIF localization and condensed nuclei in TRIP12- depleted cells was indeed observed (Fig. 2e). Inhibition of PARP activity under these conditions rescued the observed AIF translocation, demonstrating that AIF release was indeed PARylation dependent (Fig. 2e). By limiting the cellular PARylation capacity TRIP12 thus affects multiple outcomes of PAR induction in response to genotoxic stress.

### Example 3: Cooperation between a PAR-bindinq WWE domain and a HECT-type ubiquitin ligase domain enable PAR-taraeted ubiquitylation

Next, the inventors aimed to elucidate the mechanism of PARP1 regulation by TRIP12. They found TRIP12 to interact with PARP1, and this interaction was strongly impaired by PARPi (Fig. 3a). Consistently, a PARylation-defective mutant (PARP1 E988K) showed reduced binding to TRIP12 compared to wild-type PARP1 (Fig. 9a). As TRIP12 contains a putative PAR-binding WWE domain, the inventors expressed and purified the TRIP12 WWE domain (amino acids 797-911) as GST-fusion protein, and tested whether it would directly interact with auto-PARylated PARP1. As additional specificity control a mutant version was generated containing a single amino acid exchange (TRIP12 WWE R869A), which based on sequence alignment with other WWE domains27 would be predicted to abolish PAR binding. These in vitro interaction experiments revealed that the TRIP12 WWE domain indeed bound PARP1, and that the interaction was strongly dependent on PARP1 auto-PARylation (Fig. 3b). Consistently, the purified wild-type WWE domain of TRIP12, but not the R869A mutant, precipitated PARylated proteins, including PARP1, from H2O2- treated cell extracts (Fig. 3c). Moreover, in co-immunoprecipitation experiments, the interaction between TRIP12 and PARP1 was reduced for the TRIP12 WWE domain mutant R869A (Fig. S3b).

To directly test whether TRIP12 functions as PAR-targeted ubiquitin ligase for PARP1, the ubiquitylation reaction was reconstituted in vitro using purified E1 and E2 enzymes, auto-PARylated PARP1, and immune-purified full-length TRIP12. While wild-type TRIP12 was indeed able to ubiquitylate PARP1, neither the TRIP12 WWE mutant (R869A), nor a catalytically inactive mutant containing a single amino acid exchange in the HECT ubiquitin ligase domain (C2034A), were able to modify PARP1 (Fig. 3d). Importantly, also in vivo TRIP12 wild-type, but not the WWE (R869A) and HECT domain (C2034A) mutants, triggered PARP1 ubiquitylation (Fig. 3e). Thus, TRIP12 catalyzes PARP1 ubiquitylation in a PAR-dependent manner.

For the RING-type ubiquitin ligase RNF146/lduna an allosteric mechanism of PAR-dependent activation was recently described28, and the inventors therefore reasoned that a similar mechanism might be at work for the HECT-type ubiquitin ligase TRIP12. Indeed, addition of purified PAR chains greatly stimulated the enzymatic activity of TRIP12, and this effect was abolished when the PAR-binding-deficient WWE mutant (R869A) was used (Fig. 3f). Taken together, the in vitro and in vivo results thus provide biochemical support for an allosteric mechanism to activate TRIP12 in a PAR-binding- and WWE-domain-dependent manner. With analogous mechanisms being employed by both RING-type (RNF146/lduna) and HECT-type (TRIP12) ubiquitin E3 ligases, PAR-binding-mediated allosteric activation seems to be a general mechanism for activation of and target protein recognition by WWE- containing ubiquitin E3 ligases.

In line with the ubiquitylation data, over-expression of wild-type TRIP12 reduced PARP1 levels, while over-expression of the WWE (R869A) and HECT domain (C2034A) mutants had no effect (Fig. 4a). Conversely, depletion of TRIP12 resulted in reduced ubiquitylation of PARP1 in cells (Fig. 4b), and stabilized PARP1 in cycloheximide (CHX) chase experiments (Fig. 4c). Similarly, inhibition of PAR formation by the PARPi PJ-34 stabilized PARP1 levels, and additional depletion of TRIP12 did not result in a further increase in PARP1, consistent with PAR-dependent turnover of PARP1 by TRIP12 (Fig. 4d and 10a). Reassuringly, the elevated PARP1 abundance upon TRIP12 depletion was rescued by siRNA-resistantwild-type TRIP12, but not by the catalytically inactive C2034A mutant or by the PAR binding- deficient R869A mutant (Fig. 4e), despite all TRIP12 versions being expressed at similar levels (Fig. 10b). Moreover, short-term inhibition of the proteasome stabilized PARP1 upon TRIP12 over-expression (Fig. 4f), and comparable effects were observed upon PARPi (Fig. 4g), in agreement with PAR-dependent, proteasome- mediated degradation of PARP1. Finally, the inventors also found TRIP12 to be recruited to sites of DNA damage in a PAR-, PARP1- and WWE-domain-dependent manner (Fig. 10c,d), indicating that TRIP12 not only controls basal PARP1 levels but also participates in PAR-triggered protein ubiquitylation in response to genotoxic stress and chromosome breakage. Taken together, the in vitro and in vivo data reveal that through its PAR-binding WWE domain and the carboxy-terminal HECT domain TRIP12 functions as PAR-targeted ubiquitin ligase, which controls PARP1 abundance and activity.

### Example 4: TRIP12 constrains PARP1 trapping and extenuates PARPi-induced genotoxicity

As PARP1 is the major target of PARPi, and given that PARPi-induced cancer cell death has been associated with cytotoxic PARP1 trapping, entailing replication problems during S-phase progression and DNA damage, the inventors wondered whether TRIP12 might impact this important aspect of PARPi action. Loss of TRIP12 indeed resulted in enhanced chromatin association of PARP1 (in unchallenged conditions and more pronounced upon PARPi and when PARPi was combined with alkylation damage by methyl methanesulfonate, MMS), both when quantifying the detergent-resistant pool of PARP1 in hundreds of individual cells by high-content microscopy and also in biochemical fractionation assays (Fig. 5a,b and 11a,b). Consistent with elevated PARP1 trapping upon impaired TRIP12 function, an increase in DNA damage was obverved signaling marked by γH2AX, primarily in the S- and G2-phase of the cell cycle (Fig. 5c and 11c). DNA damage signaling upon PARPi is associated with impaired S-phase progression and reduced mitotic entry, and the inventors observed a more pronounced PARPi-evoked accumulation of cells in G2 upon TRIP12 depletion, compared to control cells (Fig. 5d). Importantly, both the increased DNA damage signaling measured by γH2AX formation as well as the more pronounced cell cycle arrest were rescued by PARP1 co-depletion (Fig. 5d,e), indicating that these signs of genotoxic stress were caused by catalytically inactivated PARP1. Also additional markers of PARPi-induced genotoxic stress, such as replication-associated formation of 53BP1 and RAD51 foci, were elevated in TRIP12-depleted cells, and PARP1 co-depletion alleviated this effect (Fig. 5e,f). Collectively, these results provide evidence that TRIP12, by limiting PARP1 abundance and PARP trapping, extenuates PARPi-induced DNA damage.

### Example 5: Down-regulation of TRIP12 sensitizes cancer cells to PARPi and is associated with elevated PARP1 abundance in cancer patients

Finally, to directly test whether TRIP12 status determines cancer cell survival upon PARPi exposure, the inventors performed colony formation assays, both in BRCA-proficient and BRCA-mutant cancer cells. Remarkably, TRIP12 down-regulation greatly sensitized BRCA-proficient cancer cells to the PARPi Olaparib (Lynparza®), and the hyper-sensitization was strictly dependent on the presence of PARP1, as PARP1 co- depletion rescued clonogenic survival in all cases (Fig. 6a-d). Very similar results were obtained with a second siRNA against TRIP12 (Fig. 12a) and with two different clinically approved PARPi, Rucaparib (Rubraca®) and Talazoparib (Talzenna®) (Fig. 12b,c). Consistent with the hyper-sensitization to PARPi originating from supra- physiological PARP1 accumulation, TRIP12 depletion had no effect on the survival of PARP1 knockout cells (Fig. 12d). These results are consistent with the notion that PARP1, locked in a catalytically impaired conformation, is the major contributor for PARPi-induced cell death in BRCA-proficient cells, and suggest that variations in PARP1 protein levels directly impact PARPi sensitivity. Also in the breast cancer cell lines MDA-MB-436 and SUM149PT, both carrying *BRCA1* mutations, TRIP12 depletion resulted in a PARP1-dependent sensitization to PARPi (Fig. 12e,f). Without wanting to be constrained by theory, the inventors therefore conclude that TRIP12 constrains PARPi efficiency both in HDR-proficient and in BRCA-mutant cells.

As the findings presented herein suggest TRIP12 expression as indicator of PARP1 stability (and thus PARP1 abundance and consequently PARPi sensitivity), the inventors explored the NCI/NIH CPTAC Data Portal (Ellis, M. J. et al. Cancer discovery 3, 1108-1112, 2013; Edwards, N. J. et al. Journal of proteome research 14, 2707-2713, 2015) for studies providing matched genotyping, gene expression and protein abundance data. They identified such matched multiomics data for cohorts of 102 breast cancer patients and 145 ovarian cancer patients. In both cohorts, TRIP12 expression showed a strong negative correlation with PARP1 protein abundance (p = 1.61 x 10⁻⁵ for the breast cancer cohort, p = 7.93 x 10⁻⁵ for the ovarian cancer cohort, Fig. 6e,f and 13a-f), fully consistent with our mechanistic and functional data. Further in line with the results obtained in cancer cell lines, BRCA status did not inform about PARP1 abundance in the patient cohorts, while the negative correlation between TRIP12 expression and PARP1 abundance held true irrespective of BRCA status (Fig. 13c-f). In summary, the findings establish TRIP12 as a PAR-targeted ubiquitin ligase that constrains PARP1 abundance and PARPi efficiency, and suggest that TRIP12 status can predict PARPi sensitivity beyond BRCA mutations (Fig. 6g,h).

As the results presented herein suggest that PARP1 levels are positively correlated with PARPi sensitivity, the inventors performed a proof-of-concept experiment with a cell line for controlled (doxycylin-induced) over-expression of PARP1 and measured PARPi response. Doxycyclin-induced over-expression indeed resulted in sensitization to PARPi (Fig. 14a), even though the over-expression in this system was moderate, i.e. 50% increased PARP1 expression compared to the control (Fig. 14b).

Given that a 50% increase in PARP1 expression was already sufficient to yield PARPi hypersensitivity, the inventors aimed to determine a threshold of TRIP12 expression, which would be sufficient for PARP1 stabilization and sensitization to PARPi. Using standard conditions, TRIP12 was reduced to around 20% of control levels, as measured by qPCR (Fig. 7g), and this resulted in pronounced PARPi sensitization (Fig. 6a-d). When TRIP12 was gradually depleted by siRNA, it was observed that reduction of TRIP12 to 40-50% of control levels, as measured by qPCR or Western blot (Fig. 15a, b), was sufficient to sensitize to PARPi (Fig. 15c). Reduced TRIP12 expression or activity to 50% of control levels is therefore sufficient to sensitize cells to PARPi, and a further reduction to 20% of control levels or below results in more severe sensitization to PARPi.

Collectively, the findings suggest that interfering with TRIP12 functions (e.g. by nucleic acid agents such as siRNA, sgRNA or antisense oligonucleotides) has beneficial effects on PARPi treatment. Chemical inhibitors against ubiquitin E3 ligases and deubiquitylating enzymes are currently in development, and one compound, Heclin, was recently described to inhibit the ubiquitin ligases Smurf2, Nedd4, and WWP2 (Mund et al. PNAS. 2014 111(47): 16736-16741). As proposed HECT ubiquitin ligase inhibitor, Heclin indeed inhibited TRIP12 activity in vitro (Fig. 16a) and in vivo (Fig. 16b). Moreover, Heclin treatment of cells resulted in elevated PARP1 levels, in a time- and dose-dependent manner (Fig. 16c). Importantly, Heclin treatment sensitized cancer cells to PARPi in clonogenic survival assays (Fig. 16d), suggesting that the combination of PARPi with Heclin (Fig. 16e), or derivatives thereof, or similar compounds targeting protein turnover, in particular of PARP1, may have beneficial effects in for PARPi therapy.

### Methods

### Cell culture, siRNA and plasmid transfections, drug treatments

Human U-2 OS cells (ATCC), RPE-1 (ATCC), HEK293T (ATCC), MCF-7 (kindly provided by Ross Chapman), MDA-MB-436 (kindly provided by Michael Hottiger), U-2 OS PARP1 KO cells (kindly provided by Keith Caldecott) and HeLa Kyoto PARP1-LAP cells stably expressing EGFP-tagged PARP1 from its natural promoter (kindly provided by Stoyno S. Stoynov) were grown under standard sterile cell culture conditions (37°C, humidified atmosphere, 5% CO2) in Dulbecco's modified Eagle's medium (DMEM, Thermo Fisher Scientific) containing 10% fetal bovine serum (GIBCO) and 1% of penicillin-streptomycin antibiotics. HCC1143 cells (kindly provided by Mohamed Bentires-Alj) were grown in RPMI-1640 medium (Thermo Fisher Scientific) containing 10% fetal bovine serum (GIBCO) and 1% penicillin- streptomycin antibiotics. SUM149PT (generated by the Ethier laboratory; available at http://www.bioivt.com/ and kindly provided by Alessandro Sartori) were grown in Ham's F-12 medium (Thermo Fisher Scientific) containing 10% fetal bovine serum and 1% of penicillin-streptomycin antibiotics. All cultured cells were routinely tested for mycoplasma contamination and assessed by quantitative image-based cytometry (QIBC) for proper proliferation and absence of abnormal stress signals. The following compounds were used at the indicated final concentrations: PJ-34 (10 µM, ALX-270-289-M001, Enzo Life Science), Talazoparib (6.25-50 nM, BMN673, Selleckchem), Olaparib (1-10 µM, AZD-2281, Selleckchem), hydrogen peroxide (0.02-0.5 mM, H3420, Sigma-Aldrich), Methyl methanesulfunate, MMS (0.01%, 129925, Sigma-Aldrich), PARG inhibitor (10 µM, PDD00017273, Tocris), FK866 (1 µM, F8557, Sigma-Aldrich), MG132 (10 µM, M7449, Sigma-Aldrich), cycloheximide (50 µM, C7698, Sigma-Aldrich).

### Edu labeling

For pulsed EdU (5-ethylnyl-2'-desoxyuridine, Thermo Fisher Scientific) incorporation, cells were incubated for 20 minutes in medium containing 10 µM EdU. The Click-iT EdU Alexa Fluor Imaging Kit (Thermo Fisher Scientific) was used for EdU detection.

### siRNA and plasmid transfections

Transfections with siRNAs were performed for 72h, unless stated otherwise, with Ambion Silencer Select siRNA using Lipofectamine RNAi/MAX (Thermo Fisher Scientific). For individual transfections the siRNAs were used at a final concentration of 25nM. When multiple siRNAs were combined, the final concentration was kept constant in all samples of an experiment. The following Ambion Silencer Select siRNAs were used: TRIP12 (s17808, s17809, s17810), PARP1 (s1098), DTX1 (s4355, s4356), DTX2 (s41519, s223231), DTX4 (s23319, s23321), HUWE1 (s19596, s19597), RNF146 (s37821, s37822). Negative Silencer Select control Neg1 from Ambion was used as a non-targeting control (siControl).

Plasmid transfections were performed using Lipofectamine 3000 (Thermo Fisher Scientific) or Fugene HD (Promega) according to the manufacturer's recommendations. Calcium phosphate transfection was used for HEK293T cells. Unless stated otherwise, cells were analyzed 24h after transfection.

### Antibodies

GFP (rabbit, TP401, Torrey Pines Biolabs), TRIP12 (rabbit, A301-814A, Bethyl), Tubulin (mouse, T6199, Sigma), PARP1 (rabbit, ab227244, Abcam), PARP1 (H-250) (rabbit, sc-7150, Santa Cruz), c-Myc (mouse, sc-40, Santa Cruz), Poly(ADP-ribose) (rabbit, ALX-210-890A-0100, Enzo), γH2AX (mouse, 613402, BiolLegend), γH2AX (rabbit, 9718, Cell Signaling), AIF (rabbit, sc-5586, Santa Cruz), FLAG (mouse, F3165, Sigma), Ub P4D1 (mouse, sc-8017, Santa Cruz), HA.11 (mouse, 901513, BioLegend), H3 (rabbit, ab1791, Abcam), 53BP1 (mouse, MAB3802, Merk Millipore), RAD51 (rabbit, 70-002, BioAcademia), XRCC1 (mouse, H00007515- B01P, Novus Biologicals).

### Immunostaininq

For standard immunofluorescence stainings, high-content microscopy and QIBC analyses, cells were seeded on sterile 12 mm glass coverslips and allowed to proliferate until they reached a cell density of 70-90%. Cells were then fixed in 3% formaldehyde for 15 minutes at room temperature, washed once in PBS, permeabilized for 5 minutes at room temperature in 0.2% Triton X-100 (Sigma- Aldrich) in PBS, washed twice in PBS and incubated in blocking solution (filtered DMEM containing 10% FBS and 0.02% Sodium Azide) for 15 minutes at room temperature. When the staining was combined with an EdU Click-iT reaction, this reaction was performed prior the incubation with primary antibody according to manufacturer's recommendations (Thermo Fisher Scientific). Where indicated, cells were pre-extracted in 0.2% Triton X-100 in PBS for two minutes on ice prior to formaldehyde fixation. All primary antibodies were diluted in blocking solution and incubated for 2h at room temperature. Secondary antibodies (Alexa Fluor 488, 568, 647 anti-mouse and anti-rabbit IgG from Thermo Fischer Scientific) were diluted 1:500 in blocking solution and incubated at room temperature for 1h. Cells were washed once with PBS and incubated for 10 minutes with 4',6-Diamidino-2-Phenylindole Dihydrochloride (DAPI, 0.5 µg/ml) in PBS at room temperature. Following three washing steps in PBS, coverslips were briefly washed with distilled water and mounted on 5 µl Mowiol-based mounting media (Mowiol 4.88 (Calbiochem) in Glycerol/TRIS).

### High-content microscopy and quantitative image-based cytometry (QIBC)

Automated multichannel wide-field microscopy for high-content imaging and quantitative image-based cytometry (QIBC) was performed using the Olympus ScanR System as described previously (Kilic, S. et al. EMBO J 38, e101379, 2019). Images were analyzed with the inbuilt Olympus ScanR Image Analysis Software Version 3.0.1, a dynamic background correction was applied, and nuclei segmentation was performed using an integrated intensity-based object detection module based on the DAPI signal. All downstream analyses were focused on properly detected nuclei containing a 2C-4C DNA content as measured by total and mean DAPI intensities. Fluorescence intensities were quantified and are depicted as arbitrary units. Color-coded scatter plots of asynchronous cell populations were generated with Spotfire data visualization software (TIBCO) and GraphPad Prism 8.0 was used for depicting data as violin plots. Within one experiment, similar cell numbers were compared for the different conditions. For visualizing discrete data in scatter plots, mild jittering (random displacement of data points along discrete data axes) was applied in order to demerge overlapping data points. Representative scatter plots and quantifications of independent experiments, typically containing several thousand cells each, are shown.

### Laser micro-irradiation

Cells were grown on 12 mm coverslips and pre-sensitized with 10 µM 5'-bromo-2-deoxyuridine (BrdU) for 24 hours prior to laser micro-irradiation with a pulsed UV-A laser. Laser tracks were visualized on a Leica DMI 6000 inverted microscope using an HCX Plan APO DIC 63x oil objective (1.4-0.6 NA) and analyzed using Fiji. Briefly, the average pixel intensity of laser tracks was measured within the locally irradiated area (I_{damage}), in the nucleoplasm outside the locally irradiated area (I_{nucleoplasm}) and in a region not containing cells in the same field of view (I_{background}). The level of protein accumulation relative to the protein level in the nucleoplasm was calculated as follows: (I_{damage} - I_{background})/(I_{nucleoplasm} - I_{buckground}).

### RNA extraction, reverse transcription and quantitative PCR

RNA was purified with TRIzol reagent (Life Technologies), primed with random hexamers (11034731001, Roche) and reverse-transcribed to cDNA using a MultiScribe Reverse Transcriptase (4311235, Thermo Fisher). Quantitative PCR (qPCR) was performed with KAPA SYBR FAST qPCR Kit (KAPA Biosystems) on a Rotor Gene Q system (Qiagen). Samples were run in triplicates, normalized to RPS12, and the results are depicted as relative fold changes. The following primers pairs were used:
(SEQ ID 001): hTRIP12 F: 5'-GGATGCTGTGAGCAGAGAGA-3'
(SEQ ID 002): hTRIP12 R: 5'-CATCTGATCCATCGTCATCG-3'
(SEQ ID 003): hPARP1 F: 5'-GCATCAGCACCAAAAAGGAGGTGG-3'
(SEQ ID 004): hPARP1 R: 5'-GATTTGTTGATACCTTCCTCCTTGACCTGG-3'
(SEQ ID 005): hRPS12 F: 5'-GGAGGCTTGGGTGCGTTCAAG-3'
(SEQ ID 006): hRPS12 R: 5'-GGTGGCAGTTTTGTTCCGGTTGC-3'

### Plasmids and constructs

Cloning was done using chemically competent bacteria generated in-house, derived from Library Efficency™ DH5a™ Competent Cells (Thermo Fisher Scientific). Correct cloning, integration into target vectors and mutagenesis was confirmed by DNA sequencing.

### Cloning of GFP-taaaed TRIP12

The full-length TRIP12 (TRIP12-203, transcript ENST 00000389044.8) was cloned into the pAc-GFP1-C1 (Clontech) vector using the following primers: TRIP12-F- Kpnl 5'-AATAGGTACCATGTCCAACCGGCCTAATAAC-3' (SEQ ID 007) and TRIP12-R-Xmal 5'-AATACCCGGGCCTTAGGAAAGATGGAACGACTG-3' (SEQ ID 008). In order to generate TRIP12 with a complete WWE domain (derived from TRIP12-204, transcript ENST 00000389045.7) the following stretch of 27 amino acids was introduced into the TRIP12-203 WWE domain between aa832 and aa833 (AAHQVGEDEISLSTLGRVYTIDFNSMQ) (SEQ ID 009). The 27 aa stretch was synthesized as DNA fragment by Integrated DNA technologies (IDT). The synthesized DNA fragment contained at 5' Pvul and 3' Nrul restriction sites. In order to introduce the synthesized fragment into the pAcGFP1-C1 TRIP12-203 the following primers were used to generate silent mutations in the TRIP12 DNA at the site of the insertion between aa832 and aa833: Pvul-F 5'- GCGATATGGCAGTGGCGTGACGATCGGGGCCTCTGGC-3' (SEQ ID 0010). and Nrul-F 5'- GAGAAAACCTAACCCGTTCGCGAATAGTAACACTAGTGG-3' (SEQ ID 0011). Mutation sites were introduced by site directed mutagenesis using Phusion HSII Polymerase (Thermo Scientific). The amino acid substitution C2034A and R869A were generated by site-directed mutagenesis by site direct mutagenesis with the following primers: TRIP12 C2034A-F 5'-GCCCTCTGTAATGACTGCTGTGAACTATCTTAA-3' (SEQ ID 012) and TRIP12 C2034C-R 5'-CTTAAGATAGTTCACAGCAGTCATTACAGAGGGC-3' (SEQ ID 013) and TRIP12 R869A-F 5'-CACGGGAACAGCAGCTGCCATTCAGAGAAAACC-3' (SEQ ID 0014) and TRIP12 R869A-R 5'-GGTTTTCTCTGAATGGCAGCTGCTGTTCCCGTG-3' (SEQ ID 015). The siRNA resistant mutant of the pAc-GFP1-C1 TRIP12 constructs were generated by site directed mutagenesis using the following primers: siRNA res 6x-F 5'CTAGGCAATTCGACTCATTTAGAGATGGATTTGAATCAGTCTTCC-3' (SEQ ID 016) and siRNA res 6x-R 5'- CCATCTCTAAATGAGTCGAATTGCCTAGAAACGCCTTC- 3' (SEQ ID 0017).

### Cloning of FLAG-taaaed TRIP12

The pcDNA 3.1 FLAG TRIP12 WT, C2034A and R869A were generated by Gibson assembly with the following primers with homology to the pcDNA 3.1 FLAG vector: pcDNA T12 GA -F 5'-CGGATCCACTAGTCCAGTGTGGTGGATGTCCAACCG GCC-3' (SEQ ID 018) and pcDNA T12 GA-R 5'GGTTTAAACGGGCCCTCTAGACTCGAGCTCAGGAAAGATGGAACG-3' (SEQ ID 019). The primers to generate linearize pcDNA 3.1 FLAG were the following: pcDNA GA lin-F 5'- GCTCGAGTCTAGAGGGCCCGTTTAAACC-3' (SEQ ID 020) and pcDNA GA lin-R 5'- CCACCACACTGGACTAGTGGATCCG-3' (SEQ ID 021).

### Cloning of GST-TRIP12-WWE domain WT and R869A mutant

TRIP12-WWE domain WT and R869A were generated amplifying by PCR the WWE domain of the previously generated GFP-TRIP12-203. The following primers were used: TRIP12 WWE EcoRI-F5'-TATATAGAATTCATGTTGAAGAAGGGAAATGCAC-3' (SEQ ID 022) and TRIP12 WWE Eagl-R 5'- ACGTCGGCCGTTACTTAATAAAA GACTTAGCCAGTTC-3' (SEQ ID 023). pGEX 6P-1 was linearized with EcoRI and Eagl for 2h at 37°C, followed by dephosphorylation with 10 units of Calf intestinal phosphatase (NEB) for 1 hour at 37°C. The PCR amplified WWE domain was digested with EcoRI and Eagl for 2h at 37°C. Both fragments were gel purified and ligated using Rapid Ligation Kit (Roche).

### TRIP12 purification from HEK293T cells

HEK293T cells were transfected with plasmids encoding FLAG-TRIP12 WT or the point mutants R869A or C2034A. Cells were lysed in TNE buffer (50 mM Tris-HCI pH 8.0, 150 mM NaCl, 0,1% Igepal CA630, 1 mM EDTA) supplemented with 2 mM MgCl2, cOmplete inhibitor cocktail (Roche), phosphoSTOP (Roche) and 25 U/ml benzonase. One milligram of protein lysates was immunoprecipitated using protein G- sepharose (GE Heltcare) coupled to 1 µl of anti-FLAG antibody for 3 hours at 4°C. After four washes with IP buffer the beads were first equilibrated in TE buffer (50 mM Tris-HCI pH 8.0, 150 mM NaCl) supplemented with cOmplete inhibitor cocktail (Roche), 1 mM PMSF, and then re-suspended in 5 volumes of TE buffer with 0.3 mg/ml FLAG peptide (3x FLAG peptide, Sigma) for 1 hour at 4°C at low rpm. Samples were then centrifuged at 100 x g for two minutes and the supernatant containing the eluted protein was stored at -80°C.

### In vitro ADP-ribosylation assay with recombinant proteins

To obtain auto-PARylated PARP1, human recombinant His-tagged PARP1 was incubated in reaction buffer RB (50 mM Tris-HCI pH 8.0, 4 mM MgCl2, 150 mM NaCl, 1x cOmplete inhibitor cocktail) supplemented with 20 µM - 200 µM of NAD+ and 200 nM of double-stranded annealed 40 bp long oligomer (5'TGCGACAACGATGAGATTGCCACTACTTGAACCAGTGCGG-3') (SEQ ID 024) for 15 minutes at 37°C. Auto-PARylation was stopped by addition of Olaparib to a final concentration of 10 µM.

### In vitro ubiquitylation

Auto-PARylated PARP1 was bound to histidine beads (ProBond) for 45 minutes at 4°C. The beads were then equilibrated in reaction buffer (25 mM Tris-HCI pH 7.4, 100 mM NaCl) and equally distributed in reaction tubes. 20 µl of FLAG-TRIP12 WT or mutants were incubated with 0.3 µg human recombinant E1 Ubiquitin-activating enzyme (UBE1, E-305-025, Boston Biochem), 0.3 µg of human recombinant UBE2L3 (E2-640, Boston Biochem), and 1 µg of purified ubiquitin in 25 mM Tris- HCI pH 7.4, 100 mM NaCl at 30°C for 2h and 30 minutes at low rpm. Energy regeneration system was used to recycle ATP in the reaction (B-10, Boston Biochem). The supernatant was then collected by 100 x g centrifugation for 2 minutes and the samples were boiled at 95°C for 5 minutes after the addition of 10x SDS-PAGE loading buffer. For in vitro ubiquitylation assays to monitor TRIP12 activity in absence or presence of purified PAR chains, 20 µl of purified FLAG-TRIP12 WT or R869A mutant were incubated with 0.3 µg E1, 0.3 µg E2, 1 µg of ubiquitin, and purified PAR (4336-100-01, TREVIGEN) at 0, 0.2, or 1 µM final concentration in 25 mM Tris-HCI pH 7.4, 100 mM NaCl at 30°C for 2h and 30 minutes at low rpm. Energy regeneration system (B-10, Boston Biochem) was used in all reactions to recycle ATP. The reactions were stopped by adding 10x SDS-PAGE loading buffer and samples were boiled for 5 minutes at 95°C. Proteins were separated by SDS- PAGE (Bolt 4-12% Bis-Tris Plus, NW04122, Thermo Fisher Scientific) and transferred onto polyvinylidene difluoride (PVDF) membranes (GE Healthcare). Membranes were denatured using a solution containing 6 M guanidine hydrochloride, 20 mM Tris-HCI (pH 7.4), 1 mM PMSF, and 5 µM β-mercaptoethanol for 30 min at 4°C. After extensive washing in Tris-buffered saline (TBS), membranes were blocked in TBS buffer containing bovine serum albumin (BSA) (5%) overnight and then incubated with anti-Ub P4D1 antibody (Santa Cruz) for 1 h.

### GST pull-down assays

Expression of recombinant GST-fusion proteins was performed in BL21 (DE3) cells from pGEX-6P-1 encoding GST, GST-TRIP12-WWE WT or GST-TRIP12-WWE R869A. 4 ml of bacterial pre-culture from a single colony was inoculated into 400 ml of LB medium with 100 µg/ml ampicillin and grown at 37°C and 230 rpm until reaching OD600 of 0.6. The expression was induced with 0.5 mM IPTG for 2 hours at 37°C and 230 rpm. Bacteria were harvested by centrifugation at 5'000 x g for 20 min and resuspended in 20 ml binding buffer (50 mM Tris pH 7.4, 200 mM NaCl, 0.05% NP-40, 1 mM EDTA, 1 mM DTT, 10% glycerol, 1 mM PMSF, pepstatin, bestatin, and leupeptin 1 µg/ml each) followed by cell lysis by two passages through a French press. The supernatant was recovered by centrifugation at 25'000 x g for 30 min. The supernatant was loaded onto a pre-equilibrated 1 ml GSTrap HF column (GE Healthcare Life Sciences) and washed with 10 column volumes of binding buffer. Bound proteins were eluted with 10 column volumes of elution buffer (50 mM Tris pH 7.4, 15 0mM NaCl, 0.05% NP-40, 1 mM EDTA, 1 mM DTT, 10% glycerol, 40 mM glutathione, and 1 tablet cOmplete protease inhibitor tablet (Roche) per 50 ml buffer). Eluted fractions were analyzed by 10% SDS-PAGE. Peak fractions with the highest concentration were pooled and dialyzed against dialysis buffer (50 mM Tris pH 7.4, 150 mM NaCl, 1 mM EDTA, 1 mM DTT, 20% glycerol) and the protein concentration was determined by Bradford assay.

### In vivo ubiquitylation

HEK293T or U-2 OS cells transfected with the indicated plasmids and siRNAs were washed twice with cold PBS and directly lysed on ice with RIPA buffer (50 mM Tris- HCI pH 8.0, 150 mM NaCl, 1% Igepal CA630, 0.1% SDS, 0.1% Na-deoxycholic acid), supplemented with 2 mM MgCl2, cOmplete inhibitor cocktail (Roche), phosphoSTOP (Roche), 25 U/ml benzonase, 1 mM phenylmethylsulfonylfluoride (PMSF), and 10 mM N-ethylmaleimide (NEM). Lysates were incubated for 5 minutes at room temperature and then centrifuged at 15'000 x g for 15 minutes. 500 µg of lysates were incubated with protein G-sepharose (GE Healtcare) coupled to 1 µl of anti-HA or anti-FLAG antibody for 3 hours at 4°C. Beads were collected by centrifugation, washed four times with RIPA buffer and eluted by boiling in 10x SDS-PAGE sample buffer. Samples were subjected to SDS-PAGE for immunoblotting.

### Co-immunoprecipitation

HEK293T cells were transfected as indicated and cells were washed twice with cold PBS and directly lysed on ice in 500 µl TNE buffer (50 mM Tris-HCI pH 8.0, 150 mM NaCl, 0,1% Igepal CA630, 1 mM EDTA) supplemented with 2 mM MgCl2, cOmplete inhibitor cocktail (Roche), phosphoSTOP (Roche) and 25 U/ml benzonase. Cell lysates were incubated for 5 minutes at room temperature and then centrifuged at 15'000 x g for 15 minutes. 500 µg of lysate was then incubated with protein G- sepharose coupled to 1 µl of anti-FLAG or anti-GFP antibody for 12 hours at 4°C. Beads were collected by centrifugation, washed four times with TNE buffer and eluted by boiling in 10x SDS-PAGE sample buffer. Samples were subjected to SDS- PAGE for immunoblotting.

### Chromatin fractionation

Cell pellets were collected by scraping the cells in 1x PBS supplemented with 1x protease inhibitor cocktail (cOmplete, Roche) and split in two equal fractions: (A) for the total proteome, cells were resuspended in 1x MNase buffer (0.3 M Sucrose, 50 mM Tris pH 7.5, 30 mM KCI, 7.5 mM NaCl, 4 mM MgCl2, 1 mM CaCl2, 0.125% NP-40, 0.25% Na-Deoxycholate) supplemented with 1x protease inhibitor cocktail and 10 U of MNase for every 5 million cells. Cells were incubated for 30 min at 37°C, boiled in 1x SDS-loading buffer for 5 min, spun down at 16'000 x g for 5 min and the supernatant was collected for immunochemical assays. (B) For chromatin- bound and soluble fractions, cells were resuspended in chromatin extraction buffer (10 mM HEPES pH 7.6, 3 mM MgCl2, 0.5% Triton X-100, 1 mM DTT) supplemented with 1x protease inhibitor cocktail. Cells were rotated for 30 min at room temperature and spun down at 1'300 x g for 10 min at 4°C. The pellet was kept and the supernatant was centrifuged at 16'000 x g for 5 min at 4°C and collected again (soluble fraction). The supernatant was boiled in 1x SDS-loading buffer for 5 min, spun down at 16'000 x g for 5 min and used for immunochemical assays. The pellet of the chromatin extraction step (chromatin-bound fraction) was resuspended in MNase buffer supplemented with 1x protease inhibitor cocktail and 10 U of MNase for every 5 million cells. Cells were incubated for 30 min at 37°C, boiled in 1x SDS- loading buffer for 5 min, spun down at 16'000 x g for 5 min and the supernatant was collected for immunochemical assays. Protein amounts were quantified using the standard Bradford method before addition of the SDS-PAGE loading buffer.

### Immunoblotting

Proteins were separated by standard SDS-PAGE and transferred onto PVDF membranes. To reduce auto-PARylation of PARP1 interfering with PARP1 abundance measurements by Western blot, cell extracts were treated with purified PARG to degrade PAR for 15 minutes at 37°C after cell lysis. Membranes were blocked with 5% milk in PBS-T (PBS + 0.1% Tween-20) for one hour at room temperature and incubated with primary antibodies over night at 4°C. Membranes were then washed three times with PBS-T and incubated with HRP- conjugated secondary antibodies for one hour at room temperature, washed again three times with PBS-T, and protein signals were detected using ECLTM Western Blotting Detection Reagent (Amersham™).

### Clonogenic survival assay

Cells transfected with the indicated siRNAs were seeded at single cell density and exposed to PARPi at the indicated final concentration. All conditions were performed in triplicates. Cells were then incubated for 10 days and the number of colonies with more than 50 cells was counted after staining with crystal violet (0.5% crystal violet in 20% ethanol). GraphPad Prism was used to display clonogenic survival data.

### Cancer genomics analyses

A total of 102 breast cancer samples and 145 ovarian cancer samples, for which both transcriptomics and proteomics data are publicly available, were analyzed for correlations between transcript and protein levels45. Proteomics data were generated by the NCI/NIH Clinical Proteomic Tumor Analysis Consortium (CPTAC)31,32. Transcriptomics data were generated by the TCGA Research Network: https://www.cancer.gov/tcga. As copy number loss (CNL) is a primary confounding factor for protein expression, samples with PARP1 CNL were excluded from the analyses. For transcriptomics analyses, raw TRIP12 and PARP1 transcript signals were normalized to the total RNA signals per sample. For proteomics analyses, the unshared log ratio was used as raw value. The ovarian cancer proteome data were generated in two batches, which were merged with z-score normalization within each batch. To determine the effect of TRIP12 transcript levels on PARP1 protein levels, a two-step linear regression was used: First, a linear model was built with PARP1 protein levels as response variable and PARP1 transcript levels as predictor. The residual PARP1 protein levels from the first model were then used in the second linear model for TRIP12 transcript association.

## Claims

1. A method for determining the responsiveness of a patient's tumour disease to treatment by administration of a PARP inhibitor, said method comprising the steps of
a. determining, in a sample having been obtained from the patient,
i. a level of TRIP12 protein and/or
ii. a level of an mRNA encoding TRIP12 protein
wherein said level is designated a TRIP12 expression level
and/or
iii. the presence of a TRIP12 mutation, yielding a TRIP12 activity level,
in a TRIP12 status determination step and
b. comparing said TRIP12 expression level and/or said TRIP12 activity level with a threshold value,
c. assigning to said tumour disease a likelihood of responsiveness.

2. The method according to claim 1, wherein a high likelihood of responsiveness is assigned to said tumour disease if said TRIP12 expression level and/or TRIP12 activity level is below said threshold value.

3. The method according to claim 1 or 2, wherein the expression level is determined by quantitative PCR.

4. The method according to any one of the preceding claims, wherein the threshold is ≤50%, ≤40%, or even ≤30% of the TRIP12 expression level and/or TRIP12 activity level of a control sample.

5. The method according to any one of the preceding claims, wherein the PARP inhibitor is selected from Olaparib, Rucaparib, Talazoparib, Veliparib, Niraparib, Pamiparib, and CEP-9722, particularly wherein the PARP inhibitor is Olaparib, Rucaparib or Talazoparib.

6. The method according to any one of the preceding claims, wherein said tumour disease is selected from breast cancer, ovarian cancer, prostate cancer, pancreatic cancer, lung cancer, colon cancer, glioblastoma, leukemia and lymphoma.

7. The method according to any one of the preceding claims, wherein said tumour disease is **characterized by** a tumour having
a. presence of a somatic mutation or germ line mutation leading to gene BRCA deficiency or BRCA mutation, or HDR deficiency in said tumour, or
b. absence of a somatic mutation or germ line mutation leading to gene BRCA deficiency or BRCA mutation, or HDR deficiency in said tumour.

8. A pharmaceutical composition comprising a PARP inhibitor for use in treatment of a tumour disease, wherein the tumour disease is associated with tumour cells **characterized by** a level of TRIP12 expression lower than a threshold and/or TRIP12 activity lower than a threshold, particularly wherein the threshold is ≤50%, ≤40%, or even ≤30% of the TRIP12 expression level and/or TRIP12 activity level of a control sample.

9. A pharmaceutical composition comprising a PARP inhibitor for use in treatment of a tumour disease, wherein the pharmaceutical composition is administered before, concomitant with or after administration of
a. a ubiquitin ligase inhibitor, particularly a HECT ubiquitin ligase inhibitor, more particularly Heclin, and/or
b. a nucleic acid agent capable of decreasing or inhibiting or blocking TRIP12 expression.

10. The pharmaceutical composition for use in treatment of a tumour disease according to claim 9, wherein said tumour disease is **characterized by** a tumour having
a. presence of a somatic mutation or germ line mutation leading to gene BRCA deficiency or BRCA mutation, or HDR deficiency in said tumour, or
b. absence of a somatic mutation or germ line mutation leading to gene BRCA deficiency or BRCA mutation, or HDR deficiency in said tumour.

11. The pharmaceutical composition for use in treatment of a tumour disease according to any one of claims 8 to 10, wherein the PARP inhibitor is selected from Olaparib , Rucaparib, Talazoparib, Veliparib, Niraparib, Pamiparib and CEP-9722, particularly wherein the PARP inhibitor is Olaparib, Rucaparib or Talazoparib.

12. The pharmaceutical composition for use in treatment of a tumour disease according to any one of claims 9 to 11, wherein the nucleic acid agent is an inhibitory RNA or an antisense oligonucleotide capable of hybridizing to a TRIP12 mRNA, or a polynucleotide capable of expressing an inhibitory RNA capable of hybridizing to a TRIP12 mRNA.


SEQUENCE LISTING

<110>  University of Z\237rich

<120> Method for Determining PARP Inhibitor Responsiveness and Improving
PARP Inhibitor Therapy

<130>  uz409ep

<160>  24

<170>  PatentIn version 3.5

<210>  1
<211>  20
<212>  DNA
<213>  Artificial Sequence

<220>
<223>  primer

<400>  1
ggatgctgtg agcagagaga                                                    20


<210>  2
<211>  20
<212>  DNA
<213>  Artificial Sequence

<220>
<223>  primer

<400>  2
catctgatcc atcgtcatcg                                                    20


<210>  3
<211>  24
<212>  DNA
<213>  Artificial Sequence

<220>
<223>  primer

<400>  3
gcatcagcac caaaaaggag gtgg                                               24


<210>  4
<211>  30
<212>  DNA
<213>  Artificial Sequence

<220>
<223>  primer

<400>  4
gatttgttga taccttcctc cttgacctgg                                         30


<210>  5
<211>  21

<212>  DNA
<213>  Artificial Sequence

<220>
<223>  primer

<400>  5
ggaggcttgg gtgcgttcaa g                                                21


<210>  6
<211>  23
<212>  DNA
<213>  Artificial Sequence

<220>
<223>  primer

<400>  6
ggtggcagtt ttgttccggt tgc                                              23


<210>  7
<211>  31
<212>  DNA
<213>  Artificial Sequence

<220>
<223>  primer

<400>  7
aataggtacc atgtccaacc ggcctaataa c                                     31


<210>  8
<211>  33
<212>  DNA
<213>  Artificial Sequence

<220>
<223>  primer

<400>  8
aatacccggg ccttaggaaa gatggaacga ctg                                   33


<210>  9
<211>  27
<212>  PRT
<213>  Artificial Sequence

<220>
<223>  27 amino acids introduced into the TRIP12-203 WWE domain between
       aa832 and aa833

<400>  9

Ala Ala His Gln Val Gly Glu Asp Glu Ile Ser Leu Ser Thr Leu Gly
1               5                   10                  15


Arg Val Tyr Thr Ile Asp Phe Asn Ser Met Gln
            20                  25

<210>  10
<211>  37
<212>  DNA
<213>  Artificial Sequence

<220>
<223>  primer used to generate silent mutations in the TRIP12 DNA at the
       site of the insertion between aa832 and aa833

<400>  10
gcgatatggc agtggcgtga cgatcggggc ctctggc                              37


<210>  11
<211>  39
<212>  DNA
<213>  Artificial Sequence

<220>
<223>  primer used to generate silent mutations in the TRIP12 DNA at the
       site of the insertion between aa832 and aa833

<400>  11
gagaaaacct aacccgttcg cgaatagtaa cactagtgg                            39


<210>  12
<211>  33
<212>  DNA
<213>  Artificial Sequence

<220>
<223>  primer used for amino acid substitution C2034A and R869A by
       site-directed mutagenesis

<400>  12
gccctctgta atgactgctg tgaactatct taa                                  33


<210>  13
<211>  34
<212>  DNA
<213>  Artificial Sequence

<220>
<223>  primer used for amino acid substitution C2034A and R869A by
       site-directed mutagenesis

<400>  13
cttaagatag ttcacagcag tcattacaga gggc                                 34


<210>  14
<211>  33
<212>  DNA
<213>  Artificial Sequence

<220>
<223>  primer used for amino acid substitution C2034A and R869A by
       site-directed mutagenesis

<400>  14
cacgggaaca gcagctgcca ttcagagaaa acc                                    33


<210>  15
<211>  33
<212>  DNA
<213>  Artificial Sequence

<220>
<223>  primer used for amino acid substitution C2034A and R869A by
       site-directed mutagenesis

<400>  15
ggttttctct gaatggcagc tgctgttccc gtg                                    33


<210>  16
<211>  45
<212>  DNA
<213>  Artificial Sequence

<220>
<223>  primers used for generating  siRNA resistant mutant of the
       pAc-GFP1-C1 TRIP12

<400>  16
ctaggcaatt cgactcattt agagatggat ttgaatcagt cttcc                       45


<210>  17
<211>  38
<212>  DNA
<213>  Artificial Sequence

<220>
<223>  primers used for generating  siRNA resistant mutant of the
       pAc-GFP1-C1 TRIP12

<400>  17
ccatctctaa atgagtcgaa ttgcctagaa acgccttc                               38


<210>  18
<211>  39
<212>  DNA
<213>  Artificial Sequence

<220>
<223>  primers with homology to the pcDNA 3.1 FLAG

<400>  18
cggatccact agtccagtgt ggtggatgtc caaccggcc                              39


<210>  19
<211>  45
<212>  DNA
<213>  Artificial Sequence

<220>
<223>  primers with homology to the pcDNA 3.1 FLAG

<400>  19
ggtttaaacg ggccctctag actcgagctc aggaaagatg gaacg                45

<210>  20
<211>  28
<212>  DNA
<213>  Artificial Sequence

<220>
<223>  primers to generate linearize pcDNA 3.1 FLAG

<400>  20
gctcgagtct agagggcccg tttaaacc                                   28

<210>  21
<211>  25
<212>  DNA
<213>  Artificial Sequence

<220>
<223>  primers to generate linearize pcDNA 3.1 FLAG

<400>  21
ccaccacact ggactagtgg atccg                                      25

<210>  22
<211>  34
<212>  DNA
<213>  Artificial Sequence

<220>
<223>  primer

<400>  22
tatatagaat tcatgttgaa gaagggaaat gcac                            34

<210>  23
<211>  37
<212>  DNA
<213>  Artificial Sequence

<220>
<223>  primer

<400>  23
acgtcggccg ttacttaata aaagacttag ccagttc                         37

<210>  24
<211>  40
<212>  DNA
<213>  Artificial Sequence

<220>
<223>  for auto-PARylated

<400>  24
tgcgacaacg atgagattgc cactacttga accagtgcgg                      40